(19) Europäisches Patentamt
European Patent Office
Office européen des brevets

(11) EP 4 464 685 B1

(12) EUROPEAN PATENT SPECIFICATION

(45) Date of publication and mention of the grant of the patent:
**04.02.2026 Bulletin 2026/06**

(21) Application number: **24173049.8**

(22) Date of filing: **29.04.2024**

(51) International Patent Classification (IPC):
***C07C 1/32*** $^{(2006.01)}$ ***C07C 19/01*** $^{(2006.01)}$
***C07C 9/22*** $^{(2006.01)}$

(52) Cooperative Patent Classification (CPC):
(C-Sets available)
**C07C 19/01; C07C 1/326** (Cont.)

# (54) PROCESS FOR PREPARING 17-METHYLALKANE COMPOUND

VERFAHREN ZUR HERSTELLUNG EINER 17-METHYLALKANVERBINDUNG

PROCÉDÉ DE PRÉPARATION D’UN COMPOSÉ 17-MÉTHYLALCANE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **01.05.2023 JP 2023075367**

(43) Date of publication of application:
**20.11.2024 Bulletin 2024/47**

(73) Proprietor: **SHIN-ETSU CHEMICAL CO., LTD.**
**Tokyo 1000005 (JP)**

(72) Inventors:
- **MIYAKE, Yuki**
  **Niigata, 942-8601 (JP)**
- **WATANABE, Takeru**
  **Niigata, 942-8601 (JP)**
- **KINSHO, Takeshi**
  **Niigata, 942-8601 (JP)**

(74) Representative: **De Vries & Metman**
**Overschiestraat 180**
**1062 XK Amsterdam (NL)**

(56) References cited:
- **VAN WILGENBURG ELLEN ET AL: "Deciphering the Chemical Basis of Nestmate Recognition", JOURNAL OF CHEMICAL ECOLOGY, vol. 36, no. 7, 17 June 2010 (2010-06-17), NL, pages 751 - 758, XP093206168, ISSN: 0098-0331, DOI: 10.1007/s10886-010-9812-4**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 1/326, C07C 9/22**

## Description

TECHNICAL FIELD

The present invention relates to a process for preparing a 17-methylalkane compound. More specifically, the present invention relates to a process for preparing 17-methylpentatriacontane and 17-methylheptatriacontane among a variety of 17-methylalkane compounds, which are nest mate recognition pheromones of the Argentine ant (*Linepithema humile*), one of the world's worst 100 invasive alien species.

BACKGROUND ART

The Argentine ant (*Linepithema humile)* invades countries around the world, and significantly impacts ecosystems by forming supercolonies that displace native ants. Argentine ants also form symbiotic relationships that protect aphids and scale insects from natural enemies in return for the honeydew of these agricultural pests, often making biological control with natural enemies ineffective in regions of high Argentine ant density. Furthermore, Argentine ants are sanitary pests that invade households through slight gaps. Pesticide spraying has been used to control Argentine ants, but such traditional pesticide spraying is not very effective, and is undesirable from an environmental standpoint. Biological control methods that minimize the use of pesticides are therefore being studied, and promising pest control methods include the use of nest mate recognition pheromones (Non-Patent Literatures 1 and 2 below).

Known nest mate recognition pheromones of the Argentine ant (*Linepithema humile*) include various compounds such as 15-methylpentatriacontane; 17-methylpentatriacontane; and 5,13,17-trimethyltritriacontane, 5,13,17-trimethylpentatriacontane, and 5,13,17-trimethylheptatriacontane. Among these compounds, 5,13,17-trimethylalkane compounds such as 5,13,17-trimethyltritriacontane, 5,13,17-trimethylpentatriacontane, and 5,13,17-trimethylheptatriacontane; and 17-methylalkane compounds such as 17-methylpentatriacontane and 17-methylheptatriacontane have been found to be especially active (Non-Patent Literatures 1, 2, and 3 below). Among these compounds, the following description will focus on 17-methylalkane compounds.

A process for synthesizing a 17-methylalkane compound, 17-methylpentatriacontane, has been reported (Non-Patent Literature 2 below) in which a total yield of 37.32% is obtained in the following three steps. 2-Octadecanone, for example, is reacted with octadecylmagnesium bromide to synthesize 17-methyl-17-pentatriacontanol (a first step). 17-Methyl-17-pentatriacontanol thus obtained is subjected to a dehydration reaction in benzene in the presence of an acid catalyst, p-toluenesulfonic acid, to synthesize 17-methylenepentatriacontane (a second step). 17-Methylenepentatriacontane thus obtained is then subjected to a hydrogenation reaction in diethyl ether in the presence of palladium on carbon (Pd/C) (a third step) to obtain the aforesaid 17-methylpentatriacontane (Non-Patent Literature 2).

Furthermore, a process for synthesizing a 17-methylalkane compound, 17-methylheptatriacontane, has been reported (Non-Patent Literature 2) in which a total yield of 73.63% is obtained in the following three steps. 2-Octadecanone, for example, is reacted with eicosylmagnesium bromide to synthesize 17-methyl-17-heptatriacontanol (a first step). 17-Methyl-17-heptatriacontanol thus obtained is subjected to a dehydration reaction in benzene in the presence of an acid catalyst, *p*-toluenesulfonic acid, to synthesize 17-methyleneheptatriacontane (a second step). 17-Methyleneheptatriacontane thus obtained is then subjected to a hydrogenation reaction in diethyl ether in the presence of palladium on carbon (Pd/C) (a third step) to obtain the aforesaid 17-methylheptatriacontane.

PRIOR ART

[Non-Patent Literatures]

[Non-Patent Literature 1] Neil D Tsutsui et al., BMC Biology, 2009, 7, 71.
[Non-Patent Literature 2] Neil D Tsutsui et al., J. Chem. Ecol., 2010, 36, 751-758.
[Non-Patent Literature 3] E. Sunamura et al., Insectes Sociaux, 2009, 56, 143-147.
[Non-Patent Literature 4] Dennis H. Burns et al., J. Am. Chem. Soc., 1997, 119, 2125-2133.

OBJECT OF THE INVENTION

However, the processes for preparing 17-methylpentatriacontane and 17-methylheptatriacontane in Non-Patent Literature 2 are not industrially practical due to the use of large amounts of benzene, which is extremely toxic to the human body, as a solvent. Industrialization also is difficult because the ignitable palladium on carbon is used in a diethyl ether solvent, which is a special flammable material. Moreover, as mentioned by the authors of Non-Patent Literature 2,

hydrocarbons that are homo-coupling products of Grignard reagents are produced as by-products in the aforementioned first steps. Namely, reacting 2-octadecanone with octadecylmagnesium bromide produces hexatriacontane, and reacting 2-octadecanone with eicosylmagnesium bromide produces tetracontane. In such a case, hexatriacontane (molecular weight: 506.99) is difficult to purify by distillation because its molecular weight and boiling point are near those of the target compound, 17-methyl-17-pentatriacontanol (molecular weight: 522.97). Tetracontane (molecular weight: 563.08) also is difficult to purify by distillation because its molecular weight and boiling point are near those of the target compound, 17-methyl-17-heptatriacontanol (molecular weight: 551.03). Consequently, purification by column chromatography is essential to the preparation process because the authors purify in the first steps by column chromatography instead of distillation. Purification by column chromatography is difficult to carry out on an industrial scale (more than 100 kg), making it unsuited to industrial applications.

Furthermore, the starting material, 2-octadecanone, is unsuited to industrial applications because, for example, 2-octadecanone must be synthesized separately due to the lack of industrially practical availability (of 100 kg or more); and being a solid at a room temperature (25°C), requires time and effort to place in a reactor as a solid, or when placed in the reactor as a solution, has poor ease of preparation, and may require special equipment due to the need for heating or for adding a large amount of solvent. Moreover, although it is considered that the Grignard reagents used in the first steps are prepared from magnesium and corresponding bromoalkanes, the corresponding bromoalkanes, 1-bromooctadecane and 1-bromoicosane, both are solids. However, the bromoalkanes, 1-bromooctadecane and 1-bromoicosane, must be reacted with magnesium as solutions when preparing the Grignard reagents, which reduces the ease of preparation and is unsuited to industrial applications due to the large amounts of solvents necessary for the solutions. Although the process for preparing 17-methylpentatriacontane is similar to the process for preparing 17-methylheptatriacontane, the yield of 17-methylpentatriacontane is markedly low at 37.32% for all three steps. Consequently, it is difficult to prepare the 17-methylalkane compounds with industrially practical yields in the preparation processes of Non-Patent Literature 2.

The present invention has been made in view of the aforementioned circumstances, and aims to provide a novel compound as a synthetic intermediate for efficiently preparing 17-methylalkane compounds, including 17-methylpentatriacontane and 17-methylheptatriacontane. The present invention also aims to provide a process for preparing the novel compound, and a process for preparing 17-methylalkane compounds, including 17-methylpentatriacontane and 17-methylheptatriacontane, from the novel compound.

## SUMMARY OF THE INVENTION

As a result of intensive research to overcome the aforesaid problems of the prior art, the present inventors found that a 1-halo-7-methyltricosane compound according to the present invention is a novel compound. The 1-halo-7-methyltricosane compound can be prepared inexpensively and in large amounts, and can be purified with only distillation. The present inventors also found that 17-methylalkane compounds, including 17-methylpentatriacontane and 17-methylheptatriacontane, are efficiently prepared from the 1-halo-7-methyltricosane compound. The preparation process has a high yield by subjecting the aforesaid nucleophilic reagent, 7-methyltricosyl compound, which can be easily prepared from the aforesaid 1-halo-7-methyltricosane compound, to a coupling reaction with an electrophilic alkyl reagent that is inexpensive and industrially available. The preparation process also was found to be economic and industrially viable when preparing 17-methylpentatriacontane and 17-methylheptatriacontane.

According to a first aspect of the present invention, there is provided a 1-halo-7-methyltricosane compound of the following general formula (1):

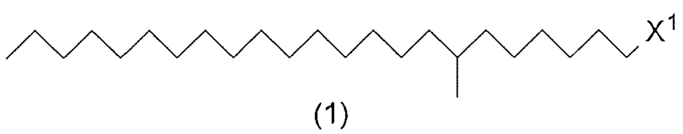

(1)

wherein $X^1$ represents a halogen atom.

According to a second aspect of the present invention, there is provided a process for preparing a 17-methylalkane compound of the following general formula (4):

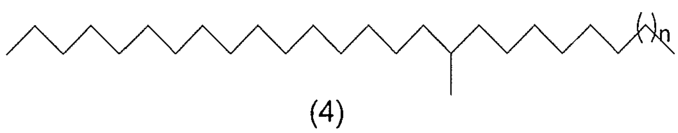

(4)

wherein *n* represents an integer of 11 to 13,

the process comprising
converting the aforesaid 1-halo-7-methyltricosane compound (1) into a nucleophilic reagent, 7-methyltricosyl compound, of the following general formula (2):

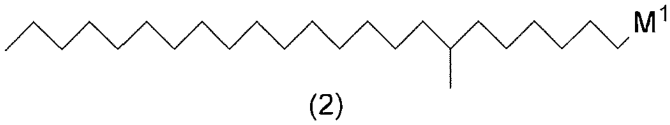

(2)

wherein $M^1$ represents Li, $MgZ^1$, $CuZ^1$, or $CuLiZ^1$, and $Z^1$ represents a halogen atom or a 7-methyltricosyl group, subsequently subjecting the aforesaid nucleophilic reagent, 7-methyltricosyl compound (2), to a coupling reaction with an electrophilic alkyl reagent of the following general formula (3):

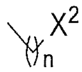

(3)

wherein $X^2$ represents a halogen atom or a *p*-toluenesulfonyloxy group, and *n* is as defined above,
to form the aforesaid 17-methylalkane compound (4).

According to a third aspect of the present invention, there is provided the process for preparing the aforesaid 17-methylalkane compound (4),

the process further comprising
subjecting a nucleophilic reagent, 2-methyloctadecyl compound, of the following general formula (5):

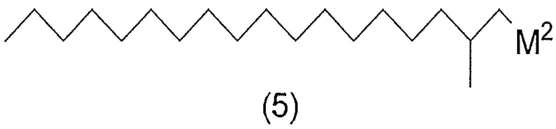

(5)

wherein $M^2$ represents Li, $MgZ^2$, $CuZ^2$, or $CuLiZ^2$, and $Z^2$ represents a halogen atom or a 2-methyloctadecyl group, to a coupling reaction with a 1,5-dihalopentane compound of the following general formula (6):

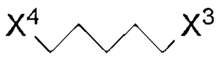

(6)

wherein $X^3$ and $X^4$ represent, independently of each other, a halogen atom,
to form the aforesaid 1-halo-7-methyltricosane compound (1). The preparation process may further comprise a purification step of purifying the 1-halo-7-methyltricosane compound (1) after the aforesaid step of forming the 1-halo-7-methyltricosane compound (1). The purification step may be carried out by only distillation.

According to a fourth aspect of the present invention, there is provided the process for preparing the aforesaid 17-methylalkane compound (4),

the process further comprising
subjecting a nucleophilic reagent, pentadecyl compound, of the following general formula (7):

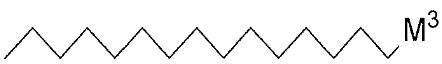

(7)

wherein $M^3$ represents Li, $MgZ^3$, $CuZ^3$, or $CuLiZ^3$, and $Z^3$ represents a halogen atom or a pentadecyl group,
to a coupling reaction with a 1,3-dihalo-2-methylpropane compound of the following general formula (8):

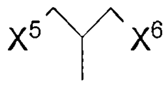

(8)

wherein $X^5$ and $X^6$ represent, independently of each other, a halogen atom,
to form a 1-halo-2-methyloctadecane compound of the following general formula (9):

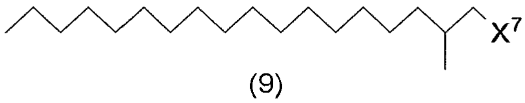

(9)

wherein $X^7$ represents a halogen atom, and
preparing the aforesaid nucleophilic reagent, 2-methyloctadecyl compound (5) from the aforesaid 1-halo-2-methyloctadecane compound (9).

According to a fifth aspect of the present invention, there is provided a process for preparing a 1-halo-7-methyltricosane compound of the following general formula (1):

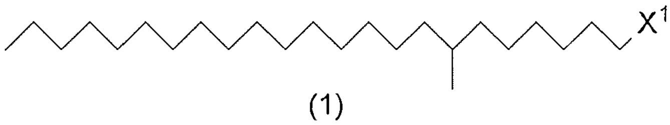

(1)

wherein $X^1$ represents a halogen atom,
the process comprising
subjecting a nucleophilic reagent, 2-methyloctadecyl compound, of the following general formula (5):

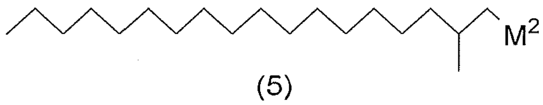

(5)

wherein $M^2$ represents Li, $MgZ^2$, $CuZ^2$, or $CuLiZ^2$, and $Z^2$ represents a halogen atom or a 2-methyloctadecyl group,
to a coupling reaction with a 1,5-dihalopentane compound of the following general formula (6):

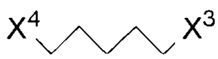

(6)

wherein $X^3$ and $X^4$ represent, independently of each other, a halogen atom,
to form the aforesaid 1-halo-7-methyltricosane compound (1). The preparation process may further comprise a purification step of purifying the 1-halo-7-methyltricosane compound (1) after the aforesaid step of forming the 1-halo-7-methyltricosane compound (1). The purification step may be carried out by only distillation.

According to the present invention, the target compounds, 17-methylalkane compounds including 17-methylpentatriacontane and 17-methylheptatriacontane, can be prepared economically and efficiently with a high yield and low environmental impact. According to the present invention, a sufficient boiling point difference can be obtained between the by-products of the steps and the aforesaid target compounds, making it possible to purify with distillation, which can be scaled up. According to the present invention, handling is easy because all of the starting materials and intermediates are liquids, making it possible to reduce the environmental impact and drastically improve the ease of preparation. Furthermore, according to the present invention, a novel synthetic intermediate useful in the preparation of the aforesaid 17-methylalkane compound can be provided.

DETAILED DESCRIPTION OF THE INVENTION

I. 1-Halo-7-methyltricosane compound (1) of the following general formula (1), and process for preparing 1-halo-7-methyltricosane compound (1)

(i) The aforesaid 1-halo-7-methyltricosane compound (1) will be described in detail below.

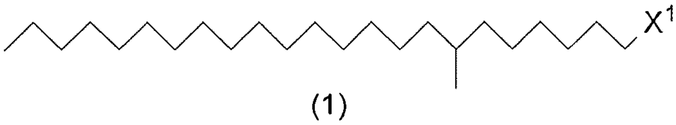

(1)

$X^1$ in the general formula (1) above represents a halogen atom. Specific examples of the halogen atom $X^1$ include a chlorine atom, a bromine atom, and an iodine atom. The halogen atom is preferably a chlorine atom and a bromine atom. By using said chlorine atom and bromine atom, a preferred melting point or solvent solubility may be ensured. The halogen atom is more preferably a chlorine atom. By using said chlorine atom, a more preferred melting point or solvent solubility may be ensured.

Specific examples of the 1-halo-7-methyltricosane compound (1) include 1-chloro-7-methyltricosane, 1-bromo-7-methyltricosane, and 1-iodo-7-methyltricosane.

The 1-halo-7-methyltricosane compound (1) is preferably 1-chloro-7-methyltricosane and 1-bromo-7-methyltricosane. By using said 1-chloro-7-methyltricosane and 1-bromo-7-methyltricosane, a preferred melting point or solvent solubility may be ensured. The 1-halo-7-methyltricosane compound (1) is more preferably 1-chloro-7-methyltricosane. By using said 1-chloro-7-methyltricosane, a more preferred melting point or solvent solubility may be ensured.

(ii) A process for preparing the aforesaid 1-halo-7-methyltricosane compound (1) will be explained in detail below.

The 1-halo-7-methyltricosane compound (1) may be prepared by, for example, subjecting a nucleophilic reagent, 2-methyloctadecyl compound, of the following general formula (5) to a coupling reaction with a 1,5-dihalopentane compound of the following general formula (6), as shown in the following chemical reaction formula:

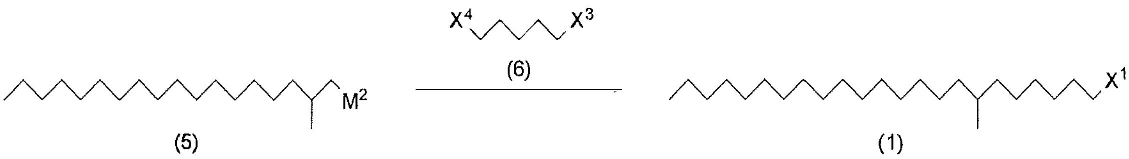

(5) (6) (1)

(iii) The aforesaid nucleophilic reagent, 2-methyloctadecyl compound (5), will be described in detail below.

In the general formula (5) above, $M^2$ represents Li, $MgZ^2$, and $CuZ^2$, and $Z^2$ represents a halogen atom or a 2-methyloctadecyl group.

Specific examples of the halogen atom $Z^2$ include a chlorine atom, a bromine atom, and an iodine atom.

Specific examples of the nucleophilic reagent, 2-methyloctadecyl compound (5), include 2-methyloctadecyllithium; 2-methyloctadecylmagnesium halide reagents (Grignard reagents) such as 2-methyloctadecylmagnesium chloride, 2-methyloctadecylmagnesium bromide, and 2-methyloctadecylmagnesium iodide; bis[2-methyloctadecyl]cuprate; and Gilman reagents such as lithium bis[2-methyloctadecyl]cuprate. The nucleophilic reagent, 2-methyloctadecyl compound (5), is preferably 2-methyloctadecylmagnesium halide reagents. By using said 2-methyloctadecylmagnesium halide reagents, a preferred ease of preparation (availability) may be ensured.

The nucleophilic reagent, 2-methyloctadecyl compound (5), may be used alone or in combination thereof, if necessary. The nucleophilic reagent, 2-methyloctadecyl compound (5), may be prepared by, for example, a preparation process that will be explained below.

(iv) The aforesaid 1,5-dihalopentane compound (6) will be described in detail below.

In the general formula (6) above, $X^3$ and $X^4$ represent, independently of each other, a halogen atom. Specific examples of the halogen atoms $X^3$ and $X^4$ include a chlorine atom, a bromine atom, and an iodine atom.

Examples of combinations of $X^3$ and $X^4$ include a chlorine atom with a chlorine atom, a bromine atom with a chlorine atom, a chlorine atom with an iodine atom, a bromine atom with a bromine atom, a bromine atom with an iodine

atom, and an iodine atom with an iodine atom.

Specific examples of the 1,5-dihalopentane compound (6) include 1,5-dichloropentane, 1,5-dibromopentane, 1,5-diiodopentane, 1-bromo-5-chloropentane, 1-chloro-5-iodopentane, and 1-bromo-5-iodopentane. The 1,5-dihalopentane compound (6) is preferably 1-bromo-5-chloropentane, 1-chloro-5-iodopentane, and 1-bromo-5-iodopentane. By using said 1-bromo-5-chloropentane, 1-chloro-5-iodopentane, and 1-bromo-5-iodopentane, a preferred yield may be ensured.

The 1,5-dihalopentane compound (6) may be used alone or in combination thereof, if necessary. The 1,5-dihalopentane compound (6) may be a commercially available one, or may be synthesized in house.

The 1,5-dihalopentane compound (6) may be synthesized by, for example, halogenating 1,5-pentanediol.

(v) The coupling reaction of the nucleophilic reagent, 2-methyloctadecyl compound (5), with the 1,5-dihalopentane compound (6) will be described in detail below.

In the coupling reaction, the amount of the nucleophilic reagent, 2-methyloctadecyl compound (5), used is preferably 0.8 to 1.4 mol, per mol of the 1,5-dihalopentane compound (6). By using said preferred amount, preferred economy may be ensured.

A solvent may be incorporated in the coupling reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N*,*N*'-dimethylpropylene urea (DMPU), hexamethylphosphoric triamide (HMPA), dichloromethane, and chloroform. The solvent is preferably hydrocarbon solvents such as toluene and xylene; ether solvents such as tetrahydrofuran, 2-methyltetrahydrofuran, and 4-methyltetrahydropyran; and acetonitrile. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. The solvent is more preferably tetrahydrofuran, 2-methyltetrahydrofuran, toluene, and xylene. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used is preferably 30 to 5,000 g, and more preferably 50 to 3,000 g, per mol of the 1,5-dihalopentane compound (6). By using said preferred amount and said more preferred amount, a preferred reactivity and a more preferred reactivity may be ensured.

The coupling reaction may be carried out in the presence of a catalyst, if necessary.

Examples of the catalyst include copper compounds such as cuprous halides such as cuprous chloride, cuprous bromide, and cuprous iodide; and cupric halides such as cupric chloride, cupric bromide, and cupric iodide. The catalyst is preferably cuprous halides. By using said cuprous halides, a preferred reactivity may be ensured. The catalyst is more preferably cuprous iodides. By using said cuprous iodides, a more preferred reactivity may be ensured.

The catalyst may be used alone or in combination thereof, if necessary. The catalyst may be a commercially available one.

The amount of the catalyst used is preferably 0.0003 to 0.3 mol, and more preferably 0.001 to 0.1 mol, per mol of the 1,5-dihalopentane compound (6). By using said preferred amount and said more preferred amount, a preferred reaction rate and/or post-treatment and a more preferred reaction rate and/or post-treatment may be ensured.

When the coupling reaction is carried out in the presence of a catalyst, a co-catalyst may be used, if necessary. Examples of the co-catalyst include phosphorus compounds such as trialkyl phosphite compounds having 3 to 9 carbon atoms such as triethyl phosphite; triarylphosphine compounds having 18 to 21 carbon atoms such as triphenylphosphine and tritolylphosphine; and arylphosphine compounds having 22 to 44 carbon atoms such as 2,2'-bis(diphenylphosphino)-1,1'-binaphthyl (BINAP). The co-catalyst is preferably trialkyl phosphite compounds. By using said trialkyl phosphite compounds, a preferred reactivity may be ensured.

The co-catalyst may be used alone or in combination thereof, if necessary. The co-catalyst may be a commercially available one.

The amount of the co-catalyst used is preferably 0.001 to 0.500 mol, and more preferably 0.005 to 0.200 mol, per mol of the 1,5-dihalopentane compound (6). By using said preferred amount and said more preferred amount, a preferred reactivity and a more preferred reactivity may be ensured.

When the coupling reaction is carried out in the presence of a catalyst, a lithium halide may be added, if necessary. Examples of the lithium halide include lithium chloride, lithium bromide, and lithium iodide. The lithium halide is preferably lithium chloride. By using said lithium chloride, a preferred reactivity may be ensured.

The amount of the lithium halide used in the coupling reaction is preferably 0.005 to 0.250 mol, per mol of the 1,5-dihalopentane compound (6). By using said preferred amount, a preferred reactivity may be ensured.

The reaction temperature of the coupling reaction varies, depending on the nucleophilic reagent, 2-methyloctadecyl compound (5), to be used, and is preferably -78 to 70°C, more preferably -20 to 50°C, and even more preferably 5 to 35°C. By using said preferred reaction temperature, said more preferred reaction temperature, and said even more preferred reaction temperature, a preferred reactivity, a more preferred reactivity, and an even more preferred reactivity may be ensured.

The reaction time of the coupling reaction varies, depending on the solvent to be used and/or the production scale, and is preferably 0.5 to 100 hours. By using said preferred reaction time, a preferred reactivity may be ensured.

When $X^3$ and $X^4$ of the general formula (6) above are different from each other, appropriate selection of the aforesaid catalyst or reaction temperature allows a more reactive halogen atom to react in the coupling reaction. When, for example, a combination of $X^3$ and $X^4$, which are different from each other in the 1,5-dihalopentane compound (6), is a combination of a chlorine atom and a bromine atom or a combination of a chlorine atom and an iodine atom, $X^1$ in the 1-halo-7-methyltricosane compound (1) would be a chlorine atom. When a combination of $X^3$ and $X^4$, which are different from each other in the 1,5-dihalopentane compound (6), are a combination of a bromine atom and an iodine atom, $X^1$ in the 1-halo-7-methyltricosane compound (1) would be a bromine atom.

In the step in which a coupling reaction for preparing the 1-halo-7-methyltricosane compound (1) is carried out, 17,20-dimethylhexatriacontane having 38 carbon atoms is by-produced as an impurity. However, the impurity and the aforesaid target compound, 1-halo-7-methyltricosane compound (1) having 24 carbon atoms, have sufficiently different boiling points. Accordingly, the aforesaid target compound can be easily separated from the aforesaid impurity by distillation, so that the 1-halo-7-methyltricosane compound (1) can be prepared with high purity.

The melting points of haloalkane compounds increase as the carbon atoms increase. For example, the melting point of 1-chlorohexadecane is 8 to 14°C, the melting point of 1-chlorooctadecane is 20°C, the melting point of 1-chloroeicosane is 37°C, and the melting point of 1-chlorodocosane is 41°C. Accordingly, 1-chlorohexadecane having up to 16 carbon atoms may be used as a liquid at a room temperature of 20 to 25°C without the need for heating and dissolving in a solvent. Among the aforesaid 1-halo-7-methyltricosane compounds (1), contrary to expectations, 1-chloro-7-methyltricosane (1: $X^1$ = Cl), which has 24 carbon atoms, was a liquid at 15°C. It is considered that the liquid state is maintained because, unlike linear haloalkanes, the methyl branch at a good position lowers the melting point. Although a process for synthesizing via the intermediate, 1-chloro-2-methyleicosane, was initially considered for synthesizing the 17-methylalkane compound, 1-chloro-2-methyleicosane was a solid at 15°C despite having the same methyl branch as 1-chloro-7-methyltricosane (1: $X^1$ = Cl) and having 21 carbon atoms, which is fewer than 1-chloro-7-methyltricosane (1: $X^1$ = Cl). Thus, introducing the methyl branch at an appropriate position in the aforesaid 1-halo-7-methyltricosane compound (1) drastically reduced the melting point to be a liquid at a room temperature, which is easy to use as an intermediate for preparing 17-methylalkane compounds.

(vi) A process for preparing the aforesaid nucleophilic reagent, 2-methyloctadecyl compound (5), will be described in detail below.

The nucleophilic reagent, 2-methyloctadecyl compound (5), may be prepared in a conventional method or a process described below.

A process for preparing a 2-methyloctadecylmagnesium halide reagent (5: $M^2$ = $MgZ^2$, wherein $Z^2$ is the aforesaid halogen atom) will be described in detail below as an example of the nucleophilic reagent, 2-methyloctadecyl compound (5).

The 2-methyloctadecylmagnesium halide reagent (5: $M^2$ = $MgZ^2$, wherein $Z^2$ is the aforesaid halogen atom) is a Grignard reagent.

The 2-methyloctadecylmagnesium halide reagent (5: $M^2$ = $MgZ^2$, wherein $Z^2$ is the aforesaid halogen atom) may be prepared by, for example, reacting a 1-halo-2-methyloctadecane compound of the following general formula (9) with magnesium in a solvent, as shown in the following chemical reaction formula:

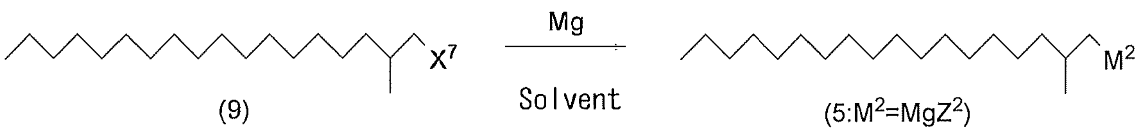


(9) (5:$M^2$=$MgZ^2$)

(vii) First, the aforesaid 1-halo-2-methyloctadecane compound (9) will be described in detail below.

$X^7$ of the general formula (9) above represents a halogen atom. Specific examples of the halogen atom $X^7$ include a chlorine atom, a bromine atom, and an iodine atom.

Specific examples of the 1-halo-2-methyloctadecane compound (9) include 1-chloro-2-methyloctadecane, 1-bromo-2-methyloctadecane, and 1-iodo-2-methyloctadecane.

The 1-halo-2-methyloctadecane compound (9) may be used alone or in combination thereof, if necessary. The 1-halo-2-methyloctadecane compound (9) may be a commercially available one, or may be synthesized in house.

The amount of magnesium used is preferably 1.0 to 2.0 gram atoms, per mol of the 1-halo-2-methyloctadecane compound (9). By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of the Grignard reagent formation may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of the Grignard reagent formation may be ensured.

The solvent may be used alone or in combination thereof, if necessary. The solvent may be a commercially available one.

The amount of the solvent used is preferably 30 to 5,000 g, and more preferably 50 to 3,000 g, per mol of the 1-halo-2-methyloctadecane compound (9). By using said preferred amount and said more preferred amount, a preferred reactivity and a more preferred reactivity may be ensured.

The reaction temperature of the aforesaid reaction with magnesium varies, depending on the solvent to be used, and is preferably 30 to 120°C. By using said preferred reaction temperature, a preferred reactivity may be ensured.

The reaction time of the aforesaid reaction with magnesium varies, depending on the solvent to be used and/or the production scale, and is preferably 0.5 to 100 hours. By using said preferred reaction time, a preferred reactivity may be ensured.

(viii) A process for preparing the aforesaid 1-halo-2-methyloctadecane compound (9) will be described in detail below.

The 1-halo-2-methyloctadecane compound (9) may be prepared in a conventional method or a process described below.

The 1-halo-2-methyloctadecane compound (9) may be prepared, for example, by subjecting a nucleophilic reagent, pentadecyl compound, of the following general formula (7) to a coupling reaction with a 1,3-dihalo-2-methylpropane compound of the following general formula (8), as shown in the following chemical reaction formula:

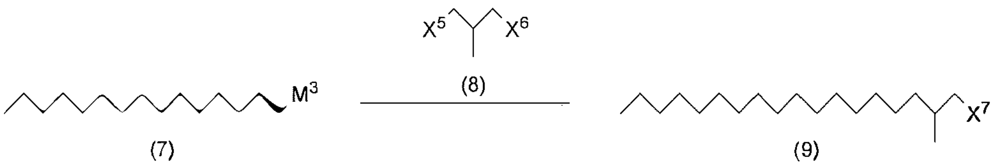

(ix) The aforesaid nucleophilic reagent, pentadecyl compound (7), will be described in detail below.

In the general formula (7) above, $M^3$ represents Li, $MgZ^3$, $CuZ^3$, or $CuLiZ^3$, and $Z^3$ represents a halogen atom or a pentadecyl group. Specific examples of the halogen atom $Z^3$ include a chlorine atom, a bromine atom, and an iodine atom and or the like. The halogen atom $Z^3$ is more preferably a bromine atom and a chlorine atom.

Specific example examples of the nucleophilic reagent, pentadecyl compound (7), include pentadecyllithium; pentadecylmagnesium halide reagents (Grignard reagents) such as pentadecylmagnesium chloride, pentadecylmagnesium bromide, and pentadecylmagnesium iodide; bis[pentadecyl]cuprate; and Gilman reagents such as lithium bis[pentadecyl]cuprate. The nucleophilic reagent, pentadecyl compound (7), is preferably pentadecylmagnesium halide reagents. By using said pentadecylmagnesium halide reagents, a preferred ease of preparation (availability) may be ensured.

The nucleophilic reagent, pentadecyl compound (7), may be used alone or in combination thereof, if necessary. The nucleophilic reagent, pentadecyl compound (7), may be a commercially available one, or may be prepared by, for example, a preparation process that will be explained below.

(x) The aforesaid 1,3-dihalo-2-methylpropane compound (8) will be described in detail below.

In the general formula (8) above, $X^5$ and $X^6$ represent, independently of each other, a halogen atom. Specific

examples of the halogen atoms $X^5$ and $X^6$ include a chlorine atom, a bromine atom, and an iodine atom.

Examples of combinations of $X^5$ and $X^6$ include a chlorine atom with a chlorine atom, a bromine atom with a chlorine atom, a chlorine atom with an iodine atom, a bromine atom with a bromine atom, a bromine atom with an iodine atom, and an iodine atom with an iodine atom.

Specific examples of the 1,3-dihalo-2-methylpropane compound (8) include 1,3-dichloro-2-methylpropane, 1,3-dibromo-2-methylpropane, 1,3-diiodo-2-methylpropane, 1-bromo-3-chloro-2-methylpropane, 1-chloro-3-iodo-2-methylpropane, and 1-bromo-3-iodo-2-methylpropane. The 1,3-dihalo-2-methylpropane compound (8) is more preferably 1-bromo-3-chloro-2-methylpropane, 1-chloro-3-iodo-2-methylpropane, and 1-bromo-3-iodo-2-methylpropane. By using said 1-bromo-3-chloro-2-methylpropane, 1-chloro-3-iodo-2-methylpropane, and 1-bromo-3-iodo-2-methylpropane, a more preferred yield may be ensured.

The 1,3-dihalo-2-methylpropane compound (8) may be used alone or in combination thereof, if necessary. The 1,3-dihalo-2-methylpropane compound (8) may be a commercially available one, or may be synthesized in house.

The 1,3-dihalo-2-methylpropane compound (8) may be synthesized by, for example, halogenating 2-methyl-1,3-propanediol.

(xi) The coupling reaction of the nucleophilic reagent, pentadecyl compound (7), with the 1,3-dihalo-2-methylpropane compound (8) will be described in detail below.

In the coupling reaction, the amount of the nucleophilic reagent, pentadecyl compound (7), used is preferably 0.8 to 1.4 mol, per mol of the 1,3-dihalo-2-methylpropane compound (8). By using said preferred amount, preferred economy may be ensured.

A solvent may be incorporated in the coupling reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N*-methylpyrrolidone (NMP), dimethyl sulfoxide (DMSO), $\gamma$-butyrolactone (GBL), acetonitrile, *N*,*N*'-dimethylpropylene urea (DMPU), hexamethylphosphoric triamide (HMPA), dichloromethane, and chloroform. The solvent is preferably hydrocarbon solvents such as toluene and xylene; ether solvents such as tetrahydrofuran, 2-methyltetrahydrofuran, and 4-methyltetrahydropyran; and acetonitrile. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. The solvent is more preferably tetrahydrofuran, 2-methyltetrahydrofuran, toluene, and xylene. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used is preferably 30 to 5,000 g, and more preferably 50 to 3,000 g, per mol of the 1,3-dihalo-2-methylpropane compound (8). By using said preferred amount and said more preferred amount, a preferred reactivity and a more preferred reactivity may be ensured.

The coupling reaction may be carried out in the presence of a catalyst, if necessary.

Examples of the catalyst include copper compounds such as cuprous halides such as cuprous chloride, cuprous bromide, and cuprous iodide; and cupric halides such as cupric chloride, cupric bromide, and cupric iodide. The catalyst is preferably cuprous halides. By using said cuprous halides, a preferred reactivity may be ensured. The catalyst is more preferably cuprous iodide. By using said cuprous iodide, a more preferred reactivity may be ensured.

The catalyst may be used alone or in combination thereof, if necessary. The catalyst may be a commercially available one.

The amount of the catalyst used is preferably 0.0003 to 0.3 mol, and more preferably 0.001 to 0.1 mol, per mol of the 1,3-dihalo-2-methylpropane compound (8). By using said preferred amount and said more preferred amount, a preferred reaction rate and/or post-treatment and a more preferred reaction rate and/or post-treatment may be ensured.

When the coupling reaction is carried out in the presence of a catalyst, the coupling reaction may be carried out in the presence of a co-catalyst, if necessary. Examples of the co-catalyst include phosphorus compounds such as trialkyl phosphite compounds having 3 to 9 carbon atoms such as triethyl phosphite; triarylphosphine compounds having 18 to 21 carbon atoms such as triphenylphosphine and tritolylphosphine; and arylphosphine compounds having 22 to 44 carbon atoms such as 2,2'-bis(diphenylphosphino)-1,1'-binaphthyl (BINAP). The co-catalyst is preferably trialkyl phosphite compounds. By using said trialkyl phosphite compounds, a preferred reactivity may be ensured.

The co-catalyst may be used alone or in combination thereof, if necessary. The co-catalyst may be a commercially available one.

The amount of the co-catalyst used is preferably 0.001 to 0.500 mol, and more preferably 0.005 to 0.200 mol, per mol of the 1,3-dihalo-2-methylpropane compound (8). By using said preferred amount and said more preferred amount, a

preferred reactivity and a more preferred reactivity may be ensured.

When the coupling reaction is carried out in the presence of a catalyst, a lithium halide may be added, if necessary. Examples of the lithium halide include lithium chloride, lithium bromide, and lithium iodide. The lithium halide is preferably lithium chloride. By using said lithium chloride, a preferred reactivity may be ensured.

The amount of the lithium halide used in the coupling reaction is preferably 0.005 to 0.250 mol, per mol of the 1,3-dihalo-2-methylpropane compound (8). By using said preferred amount, a preferred reactivity may be ensured.

The reaction temperature of the coupling reaction varies, depending on the nucleophilic reagent, pentadecyl compound (7), to be used, and is preferably -78 to 70°C, more preferably -20 to 50°C, and even more preferably 5 to 35°C. By using said preferred reaction temperature, said more preferred reaction temperature, and said even more preferred reaction temperature, a preferred reactivity, a more preferred reactivity, and an even more preferred reactivity may be ensured.

The reaction time of the coupling reaction varies, depending on the solvent to be used and/or the production scale, and is preferably 0.5 to 100 hours. By using said preferred reaction time, a preferred reactivity may be ensured.

When $X^5$ and $X^6$ are different from each other in the general formula (8) above, appropriate selection of the aforesaid catalyst or reaction temperature allows a more reactive halogen atom to react in the coupling reaction. When, for example, a combination of $X^5$ and $X^6$, which are different from each other in the 1,3-dihalo-2-methylpropane compound (8), is a combination of a chlorine atom and a bromine atom or a combination of a chlorine atom and an iodine atom, $X^7$ in the 1-halo-2-methyloctadecane compound (9) would be a chlorine atom. When a combination of $X^5$ and $X^6$, which are different from each other in the 1,3-dihalo-2-methylpropane compound (8), is a combination of a bromine atom and an iodine atom, $X^7$ in the 1-halo-2-methyloctadecane compound (9) would be a bromine atom.

In the step in which a coupling reaction for preparing the 1-halo-2-methyloctadecane compound (9) is carried out, triacontane having 30 carbon atoms is by-produced as an impurity. However, the impurity has a boiling point sufficiently different from that of the aforesaid target compound, 1-halo-2-methyloctadecane compound (9) having 19 carbon atoms. Accordingly, the aforesaid target compound can be easily separated from the aforesaid impurity with distillation, leading to the preparation of the 1-halo-2-methyloctadecane compound (9) with high purity.

As mentioned above, the melting points of haloalkane compounds increase as the carbon atoms increase. For example, 1-chlorohexadecane having up to 16 carbon atoms may be used as a liquid at a room temperature of 20 to 25°C without the need for heating and dissolving in a solvent. On the other hand, 1-chloro-2-methyleicosane that was synthesized separately and had 21 carbon atoms was a solid at 15°C, while the 1-halo-2-methyloctadecane compound (9) and 1-chloro-2-methyloctadecane having similar methyl branches were in the liquid state even at 15°C.

(xii) A process for preparing the aforesaid nucleophilic reagent, pentadecyl compound (7), will be described in detail below.

The nucleophilic reagent, pentadecyl compound (7), may be prepared in a conventional method or a process described below.

A process for preparing a pentadecylmagnesium halide reagent (7: $M^3$ = $MgZ^3$, wherein $Z^3$ is a halogen atom) as the nucleophilic reagent, pentadecyl compound (7), will be described as an example in detail below. The pentadecylmagnesium halide reagent (7: $M^3$ = $MgZ^3$) is a Grignard reagent.

The pentadecylmagnesium halide reagent (7: $M^3$ = $MgZ^3$) may be prepared by, for example, reacting a 1-halopentadecane compound of the following general formula (10) with magnesium in a solvent, as shown in the following chemical reaction formula:

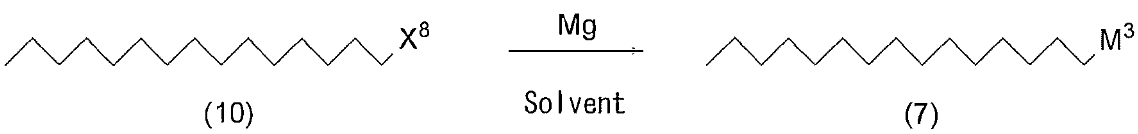


(10) (7)

First, the aforesaid 1-halopentadecane compound (10) will be described in detail below.

In the general formula (10) above, $X^8$ represents a halogen atom. Specific examples of the halogen atom $X^8$ include a chlorine atom, a bromine atom, and an iodine atom.

Specific examples of the 1-halopentadecane compound (10) include 1-chloropentadecane, 1-bromopentadecane, and 1-iodopentadecane.

The 1-halopentadecane compound (10) may be used alone or in combination thereof, if necessary. The 1-halopentadecane compound (10) may be a commercially available one, or may be synthesized in house.

The amount of magnesium used is preferably 1.0 to 2.0 gram atoms, per mol of the 1-halopentadecane compound (10). By using said preferred amount, a preferred completion of the reaction may be ensured.

Examples of the solvent include general solvents such as, for example, ether solvents such as tetrahydrofuran

(THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of the Grignard reagent formation may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of the Grignard reagent formation may be ensured.

The solvent may be used alone or in combination thereof, if necessary. The solvent may be a commercially available one.

The amount of the solvent used is preferably 30 to 5,000 g and more preferably 50 to 3,000 g, per mol of the 1-halopentadecane compound (10). By using said preferred amount and said more preferred amount, a preferred reactivity and a more preferred reactivity may be ensured.

The reaction temperature of the aforesaid reaction with magnesium varies, depending on the solvent to be used, and is preferably 30 to 120°C. By using said preferred reaction temperature, a preferred reactivity may be ensured.

The reaction time of the aforesaid reaction with magnesium varies, depending on the solvent to be used and/or the production scale, and is preferably 0.5 to 100 hours. By using said preferred reaction time, a preferred reactivity may be ensured.

II. Process for preparing 17-methylalkane compound of the following general formula (4)

One target compound of the present invention, the 17-methylalkane compound of the following general formula (4), is prepared according to the preparation process of the following chemical reaction formula:

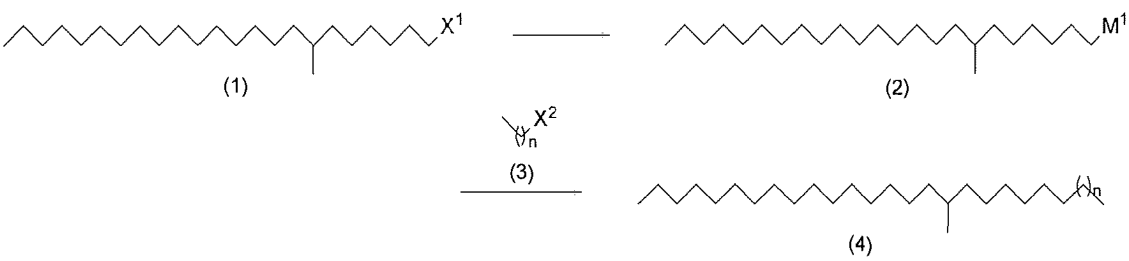

(1)

(2)

(3)

(4)

(i) First, the aforesaid nucleophilic reagent, 7-methyltricosyl compound (2), will be described in detail below. In the general formula (2) above, $M^1$ represents Li, $MgZ^1$, $CuZ^1$, or $CuLiZ^1$, and $Z^1$ represents a halogen atom or a 7-methyltricosyl group. Specific examples of the halogen atom $Z^1$ include a chlorine atom, a bromine atom, and an iodine atom.

The 7-methyltricosylmagnesium halide reagent (2: $M^1$ = $MgZ^1$) is a Grignard reagent.

Specific examples of the nucleophilic reagent, 7-methyltricosyl compound (2), include 7-methyltricosyllithium; 7-methyltricosylmagnesium halide reagents (Grignard reagents) such as 7-methyltricosylmagnesium chloride, 7-methyltricosylmagnesium bromide, and 7-methyltricosylmagnesium iodide; bis[7-methyltricosyl]cuprate; and Gilman reagents such as lithium bis[7-methyltricosyl]cuprate. The nucleophilic reagent, 7-methyltricosyl compound (2), is preferably 7-methyltricosylmagnesium halide reagents. By using said 7-methyltricosylmagnesium halide reagents, a preferred ease of preparation (availability) may be ensured.

(ii) A process for preparing the aforesaid nucleophilic reagent, 7-methyltricosyl compound (2), will be explained in detail below.

The preparation process includes at least a step of obtaining the nucleophilic reagent, 7-methyltricosyl compound (2), from the aforesaid 1-halo-7-methyltricosane compound (1).

The preparation process in which, for example, the 7-methyltricosylmagnesium halide reagent (2: $M^1$ = $MgZ^1$, wherein $Z^1$ is the aforesaid halogen atom) is the nucleophilic reagent, 7-methyltricosyl compound (2), will be described in detail below.

The 7-methyltricosylmagnesium halide reagent (2: $M^1$ = $MgZ^1$, wherein $Z^1$ is the aforesaid halogen atom) may be prepared by, for example, reacting the aforesaid 1-halo-7-methyltricosane compound (1) with magnesium in a solvent, as

shown in the following chemical reaction formula:

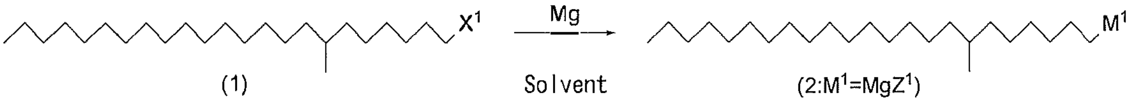


(1) (2:$M^1$=$MgZ^1$)

The 1-halo-7-methyltricosane compound (1) may be used alone or in combination thereof, if necessary.

The amount of magnesium used is preferably 1.0 to 2.0 gram atoms, per mol of the 1-halo-7-methyltricosane compound (1). By using said preferred amount, a preferred completion of the reaction may be ensured.

Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; and hydrocarbon solvents such as hexane, heptane, benzene, toluene, xylene, and cumene. The solvent is preferably hydrocarbon solvents such as toluene and xylene; and ether solvents such as tetrahydrofuran, 2-methyltetrahydrofuran, and 4-methyltetrahydropyran. By using said hydrocarbon solvents such as toluene and xylene; and ether solvents such as tetrahydrofuran, 2-methyltetrahydrofuran, and 4-methyltetrahydropyran, a preferred reaction rate of the Grignard reagent formation may be ensured. The solvent is more preferably tetrahydrofuran, 2-methyltetrahydrofuran, and 4-methyltetrahydropyran. By using said tetrahydrofuran, 2-methyltetrahydrofuran, and 4-methyltetrahydropyran, a more preferred reaction rate of the Grignard reagent formation may be ensured.

The solvent may be used alone or in combination thereof, if necessary. The solvent may be a commercially available one.

The amount of the solvent used is preferably 30 to 5,000 g, and more preferably 50 to 3,000 g, per mol of the 1-halo-7-methyltricosane compound (1). By using said preferred amount and said more preferred amount, a preferred reactivity and a more preferred reactivity may be ensured.

The reaction temperature of the aforesaid reaction with magnesium varies, depending on the solvent, and is preferably 30 to 120°C. By using said reaction temperature, a preferred reactivity may be ensured.

The reaction time of the aforesaid reaction with magnesium varies, depending on the solvent to be used and/or the production scale, and is preferably 0.5 to 100 hours. By using said preferred reaction time, a preferred reactivity may be ensured.

(iii) A process for preparing the 17-methylalkane compound (4) from the aforesaid nucleophilic reagent, 7-methyltricosyl compound (2), will be explained in detail below.

The preparation process includes at least a step of subjecting the aforesaid nucleophilic reagent, 7-methyltricosyl compound (2), to a coupling reaction with an electrophilic alkyl reagent of the following general formula (3) to form the 17-methylalkane compound (4):

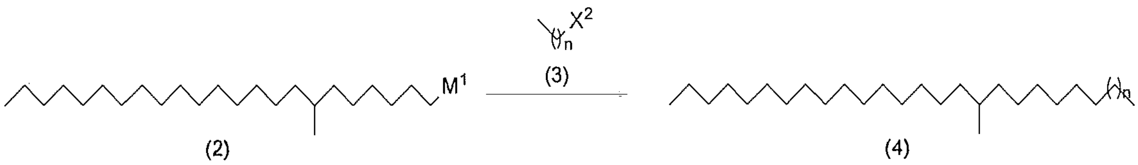


(2) (4)

The nucleophilic reagent, 7-methyltricosyl compound (2), may be used alone or in combination thereof, if necessary. The nucleophilic reagent, 7-methyltricosyl compound (2), may be a commercially available one, or may be synthesized in house.

(iv) The aforesaid electrophilic alkyl reagent (3) will be described in detail below.

In the general formula (3) above, $X^2$ represents a halogen atom or a *p*-toluenesulfonyloxy group (a $CH_3$-$C_6H_6$-$SO_2$-O(TsO) group). Specific examples of the halogen atom $X^2$ are a chlorine atom, a bromine atom, and an iodine atom. The halogen atom $X^2$ is more preferably a bromine atom and an iodine atom.

In the general formula (3) above, *n* is 11 to 13.

Specific examples of the electrophilic alkyl reagent (3) include 1-halododecane compounds (*n* = 11) such as 1-chlorododecane, 1-bromododecane, and 1-iodododecane; dodecyl *p*-toluenesulfonate *(n* = 11); 1-halotridecane compounds *(n* = 12) such as 1-chlorotridecane, 1-bromotridecane, and 1-iodotridecane; tridecyl *p*-toluenesulfonate *(n* = 12); 1-halotetradecane compounds (*n* = 13) such as 1-chlorotetradecane, 1-bromotetradecane, and 1-iodotetradecane; and tetradecyl *p*-toluenesulfonate (*n* = 13). The electrophilic alkyl reagent (3) is preferably 1-halododecane compounds (*n* =

11), 1-halotridecane compounds ($n$ = 12), and 1-halotetradecane compounds ($n$ = 13). By using said 1-halododecane compounds ($n$ = 11), 1-halotridecane compounds ($n$ = 12), and 1-halotetradecane compounds ($n$ = 13), a preferred ease of preparation (availability) may be ensured. When $n$ is 11, the 17-methylalkane compound (4) formed by subjecting the aforesaid nucleophilic reagent, 7-methyltricosyl compound (2), to a coupling reaction with the electrophilic alkyl reagent (3: $n$ = 11) wherein $n$ is 11, is one target compound of the present invention, 17-methylpentatriacontane (4: $n$ = 11). When $n$ is 13, the 17-methylalkane compound (4) formed by subjecting the aforesaid nucleophilic reagent, 7-methyltricosyl compound (2), to a coupling reaction with the electrophilic alkyl reagent (3: $n$ = 13) wherein $n$ is 13, is one target compound of the present invention, 17-methylheptatriacontane (4: $n$ = 13).

The electrophilic alkyl reagent (3) may be used alone or in combination thereof, if necessary. The electrophilic alkyl reagent (3) may be a commercially available one, or may be synthesized in house.

The electrophilic alkyl reagent (3) may be synthesized by, for example, halogenating 1-alkanol or by tosylating 1-alkanol with *p*-toluenesulfonyl chloride in the presence of a base.

(v) A coupling reaction of the nucleophilic reagent, 7-methyltricosyl compound (2), with the electrophilic alkyl reagent (3) will be described in detail below.

Similarly to the electrophilic alkyl reagent (3) wherein $X^2$ is a halogen atom, the electrophilic alkyl reagent (3) wherein $X^2$ is a *p*-toluenesulfonyloxy group may be incorporated in the aforesaid coupling reaction. This is because a *p*-toluenesulfonyloxy group is a good leaving group, similarly to a halogen atom such as a bromine atom (see, for example, Non-Patent Literature 4 above, page 2127, "Table 1", and related descriptions).

The amount of the nucleophilic reagent, 7-methyltricosyl compound (2), used in the coupling reaction is preferably 0.8 to 1.2 mol, per mol of the electrophilic alkyl reagent (3). By using said preferred amount, preferred economy may be ensured.

A solvent may be incorporated in the coupling reaction, if necessary. Examples of the solvent include general solvents such as, for example, ether solvents such as tetrahydrofuran (THF), 2-methyltetrahydrofuran (2-MeTHF), diethyl ether, dibutyl ether, 4-methyltetrahydropyran (MTHP), cyclopentylmethylether, and 1,4-dioxane; hydrocarbon solvents such as hexane, heptane, benzene, toluene, xylene, and cumene; and polar solvents such as *N*,*N*-dimethylformamide (DMF), *N*,*N*-dimethylacetamide (DMAC), *N*-methylpyrrolidone (NMP), dimethyl sulfoxide (DMSO), γ-butyrolactone (GBL), acetonitrile, *N,N'*-dimethylpropylene urea (DMPU), hexamethylphosphoric triamide (HMPA), dichloromethane, and chloroform. The solvent is preferably hydrocarbon solvents such as toluene and xylene; ether solvents such as tetrahydrofuran, 2-methyltetrahydrofuran, and 4-methyltetrahydropyran; and acetonitrile. By using said hydrocarbon solvents such as toluene and xylene; ether solvents such as tetrahydrofuran, 2-methyltetrahydrofuran, and 4-methyltetrahydropyran; and acetonitrile, a preferred reactivity may be ensured. The solvent is more preferably tetrahydrofuran, 2-methyltetrahydrofuran, toluene, and xylene. By using said tetrahydrofuran, 2-methyltetrahydrofuran, toluene, and xylene, a more preferred reactivity may be ensured.

The solvent may be used alone or in combination thereof, if necessary. The solvent may be a commercially available one.

The amount of the solvent used is preferably 30 to 5,000 g, and more preferably 50 to 3,000 g, per mol of the nucleophilic reagent, 7-methyltricosyl compound (2). By using said preferred amount and said more preferred amount, a preferred reactivity and a more preferred reactivity may be ensured.

The coupling reaction may be carried out in the presence of a catalyst, if necessary.

Examples of the catalyst include copper compounds such as cuprous halides such as cuprous chloride, cuprous bromide, and cuprous iodide; and cupric halides such as cupric chloride, cupric bromide, and cupric iodide. The catalyst is preferably cuprous halides. By using said cuprous halides, a preferred reactivity may be ensured. The catalyst is more preferably cuprous chloride. By using said cuprous chloride, a more preferred reactivity may be ensured.

The catalyst may be used alone or in combination thereof, if necessary. The catalyst may be a commercially available one.

The amount of the catalyst used is preferably 0.0003 to 0.300 mol, and more preferably 0.001 to 0.100 mol, per mol of the nucleophilic reagent, 7-methyltricosyl compound (2). By using said preferred amount and said more preferred amount, a preferred reaction rate and/or post-treatment and a more preferred reaction rate and/or post-treatment may be ensured.

When the coupling reaction is carried out in the presence of a catalyst, a co-catalyst may be used, if necessary. Examples of the co-catalyst include phosphorus compounds such as trialkyl phosphite compounds having 3 to 9 carbon atoms such as triethyl phosphite; triarylphosphine compounds having 18 to 21 carbon atoms such as triphenylphosphine and tritolylphosphine; and arylphosphine compounds having 22 to 44 carbon atoms such as 2,2'-bis(diphenylphosphino)-1,1'-binaphthyl (BINAP). The co-catalyst is preferably trialkyl phosphite compounds. By using said trialkyl phosphite compounds, a preferred reactivity may be ensured.

The co-catalyst may be used alone or in combination thereof, if necessary. The co-catalyst may be a

commercially available one.

The amount of the co-catalyst used is preferably 0.001 to 0.500 mol, and more preferably 0.005 to 0.200 mol, per mol of the nucleophilic reagent, 7-methyltricosyl compound (2). By using said preferred amount and said more preferred amount, a preferred reactivity and a more preferred reactivity may be ensured.

When the coupling reaction is carried out in the presence of a catalyst, a lithium halide may be added, if necessary. Examples of the lithium halide include lithium chloride, lithium bromide, and lithium iodide. The lithium halide is preferably lithium chloride. By using said lithium chloride, a preferred reactivity may be ensured.

The amount of the lithium halide used in the coupling reaction is preferably 0.005 to 0.250 mol, per mol of the nucleophilic reagent, 7-methyltricosyl compound (2). By using said preferred amount, a preferred reactivity may be ensured.

The reaction temperature of the coupling reaction varies, depending on the nucleophilic reagent, 7-methyltricosyl compound (2), to be used, and is preferably -78 to 70°C, more preferably -20 to 50°C, and even more preferably 5 to 35°C. By using said preferred reaction temperature, said more preferred reaction temperature, and said even more preferred reaction temperature, a preferred reactivity, a more preferred reactivity, and an even more preferred reactivity may be ensured.

The reaction time of the coupling reaction varies, depending on the solvent to be used and/or the production scale, and is preferably 0.5 to 100 hours. By using said preferred reaction time, a preferred reactivity may be ensured.

(vi) The aforesaid 17-methylalkane compound (4) will be described in detail below.

In the general formula (4) above, *n* is as defined for the general formula (3) above.

Specific examples of the 17-methylalkane compound (4) include 17-methylpentatriacontane (4: *n* = 11), 17-methylhexatriacontane (4: *n* = 12), and 17-methylheptatriacontane (4: *n* = 13).

In the step in which a coupling reaction for preparing one target compound of the present invention, 17-methylpentatriacontane (4: *n* = 11), is carried out, tetracosane having 24 carbon atoms and 17,30-dimethylhexatetracontane having 48 carbon atoms are by-produced as impurities. However, the impurities have sufficiently different boiling points from the aforesaid target compound, 17-methylpentatriacontane (4: *n* = 11) having 36 carbon atoms. The impurity, 17,30-dimethylhexatetracontane having 48 carbon atoms, has an extremely high melting point and can be easily separated because precipitation as a solid occurs on the wall of the reactor in the post-treatment of the coupling reaction without dissolving and mixing in the organic layer. Accordingly, the aforesaid target compound can be easily separated from the aforesaid impurity by distillation, column chromatography, recrystallization, or a combination thereof, leading to the preparation of 17-methylpentatriacontane (4: *n* = 11) with high purity.

Similarly, in the step in which a coupling reaction for preparing one target compound of the present invention, 17-methylheptatriacontane (4: *n* = 13), is carried out, octacosane having 28 carbon atoms and 17,30-dimethylhexatetracontane having 48 carbon atoms are by-produced as impurities. However, the impurities have sufficiently different boiling points from the aforesaid target compound, 17-methylheptatriacontane (4: *n* = 13) having 38 carbon atoms. The impurity, 17,30-dimethylhexatetracontane having 48 carbon atoms has an extremely high melting point and can be easily separated because precipitation as a solid occurs on the wall of the reactor in the post-treatment of the coupling reaction without dissolving and mixing in the organic layer. Accordingly, the aforesaid target compound can be easily separated from the aforesaid impurity by distillation, column chromatography, recrystallization, or a combination thereof, leading to the preparation of 17-methylheptatriacontane (4: *n* = 13) with high purity.

Thus, the nest mate recognition pheromone of the Argentine ant, 17-methylalkane compound (4), may be efficiently prepared from the synthetic intermediate, 1-halo-7-methyltricosane compound (1), with high ease of preparation and in fewer steps.

## EXAMPLES

The present invention will be described with reference to the following Examples. It should be noted that the present invention is not limited to or by the Examples, but the claims.

The term "purity" as used herein means an area percentage in gas chromatography (GC), unless otherwise specified. The term "product ratio" means a ratio of area percentages in GC. The term "yield" is calculated from the area percentages determined by GC.

In the Examples, monitoring of the reactions and calculation of the yields were carried out in the following GC conditions.

GC conditions: GC: Capillary gas chromatograph GC-2014 (Shimadzu Corporation); column: DB-5, 0.25 μm x 0.25 mmϕ x 30 m; carrier gas: He (1.55 mL/min), detector: FID; column temperature: 150°C, elevated in a rate of 5°C/min, and up to 230°C; column: UA-5, 0.25 μmx0.25 mmϕx10 m; carrier gas: He (1.55 mL/min), detector: FID; column temperature: 230°C, elevated in a rate of 10°C/min up to 300°C.

The yield was calculated according to the following equation in consideration of purities (% GC) of a starting material and a product.

Yield (%) = {[(weight of a product obtained by a reaction × % GC) / molecular weight of a product] ÷ [(weight of a starting material in a reaction × % GC) / molecular weight of a starting material] × 100

THF represents tetrahydrofuran, NMP represents *N*-methyl-2-pyrrolidone, and Et represents an ethyl group.

Example 1: Preparation of 1-chloropentadecane, a starting material of 1-chloro-2-methyloctadecane (9: $X^7$ = Cl)

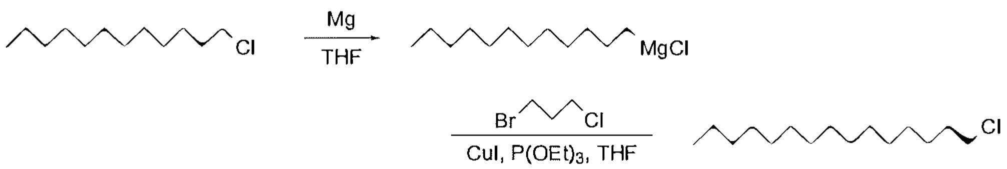


Magnesium (160.74 g, 6.61 gram atoms) and tetrahydrofuran (1,890.00 g) were placed in a reactor at a room temperature and stirred for 25 minutes at 60 to 65°C. 1-Chlorododecane (1,290.12 g, 6.30 mol, purity 100%) was then added dropwise to the reactor at 60 to 75°C. After the completion of the dropwise addition, dodecylmagnesium chloride was obtained by stirring at 75 to 80°C for 2 hours.

Next, cuprous iodide (12.00 g, 0.063 mol), triethylphosphite (25.12 g, 0.15 mol), tetrahydrofuran (630.00 g), and 1-bromo-3-chloropropane (922.44 g, 5.86 mol) were placed in another reactor, and the aforesaid dodecylmagnesium chloride that was prepared was added dropwise at 5 to 15°C. After the completion of the dropwise addition, the reaction mixture was stirred for 1 hour at 10 to 20°C. An aqueous solution of ammonium chloride (prepared from ammonium chloride (63.00 g) and water (1,735.66 g)), 20% by mass hydrochloric acid (63.00 g), and an aqueous solution (32.04 g) of 25% by mass sodium hydroxide were then added to the reaction mixture, followed by phase separation. The organic layer thus obtained was concentrated at a reduced pressure, and the resulting concentrate was distilled at a reduced pressure to obtain 1-chloropentadecane (1,311.04 g, 5.26 mol, purity 99.08%, b.p. = 150.3 to 152.3°C/0.37 kPa (2.8 mmHg)) as a liquid with a yield of 89.81%. 1-Chloropentadecane thus obtained did not include tetracosane, a homo-coupling product of dodecylmagnesium chloride (tetracosane was below the GC detection limit).

The following is the spectrum data of 1-chloropentadecane thus prepared.

Nuclear magnetic resonance spectrum: $^1$H-NMR (500 MHz, $CDCl_3$): δ = 0.88 (3H, t, J = 6.9 Hz), 1.21-1.34 (22H, m), 1.38-1.46 (2H, m), 1.77 (2H, tt, J = 6.9 Hz, 6.9 Hz), 3.53 (2H, t, J = 6.9 Hz); $^{13}$C-NMR (125 MHz, $CDCl_3$): δ = 14.1, 22.7, 26.9, 28.9, 29.4, 29.47, 29.55, 29.62, 29.65, 29.69, 31.9, 32.7, 45.2.

Mass spectrum: EI-mass spectrum (70 eV): m/z 246 ($M^+$), 217, 203, 189, 175, 161, 147, 133, 119, 105, 71, 57, 43, 29.

Infrared absorption spectrum: (D-ATR): ν = 2955, 2924, 2854, 1466, 1377, 1308, 723, 655.

Example 2: Preparation of 1-chloro-2-methyloctadecane (9: $X^7$ = Cl)

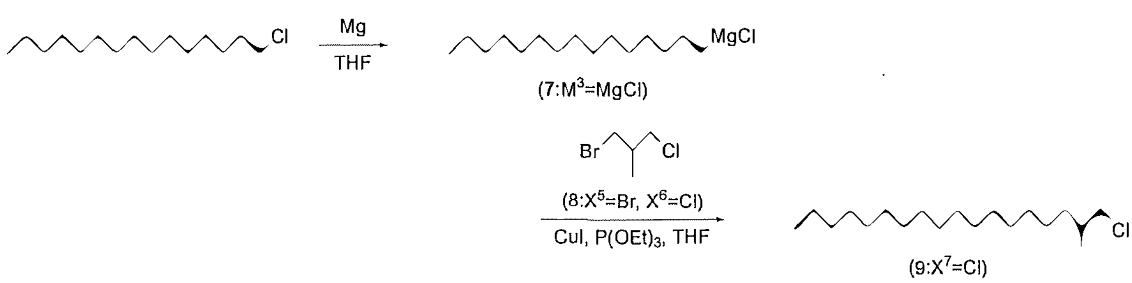


Magnesium (120.90 g, 4.98 gram atoms) and tetrahydrofuran (1,421.40 g) were placed in a reactor at a room temperature and stirred for 37 minutes at 60 to 65°C. 1-Chloropentadecane (1,180.48 g, 4.74 mol, purity 99.08%) prepared in Example 1 was then added dropwise to the reactor at 60 to 75°C. After the completion of the dropwise addition,

pentadecylmagnesium chloride (7: $M^3$ = MgCl) was obtained by stirring for 2 hours at 75 to 80°C.

Next, cuprous iodide (CuI) (9.02 g, 0.047 mol), triethyl phosphite ($P(OEt)_3$) (18.90 g, 0.11 mol), tetrahydrofuran (947.60 g), and 1-bromo-3-chloro-2-methylpropane (8: $X^5$ = Br, $X^6$ = Cl) (755.52 g, 4.41 mol, purity 100%) were placed in another reactor, and the aforesaid pentadecylmagnesium chloride that was prepared was added dropwise at 10 to 20°C. After the completion of the dropwise addition, the reaction mixture was stirred for 1.5 hours at 10 to 20°C. An aqueous solution of ammonium chloride (prepared from ammonium chloride (47.38 g) and water (1,305.32 g)), 20% by mass hydrochloric acid (90.72 g), and an aqueous solution (45.36 g) of 25% by mass sodium hydroxide were then added to the reaction mixture, followed by phase separation. The organic layer thus obtained was concentrated at a reduced pressure, and the resulting concentrate was distilled at a reduced pressure to obtain 1-chloro-2-methyloctadecane (9: $X^7$ = Cl) (1,190.20 g, 3.89 mol, purity 98.92%, b.p. = 161.1 to 167.0°C/0.044 kPa (0.33 mmHg)) as a liquid with a yield of 88.20%. 1-Chloro-2-methyloctadecane (9: $X^7$ = Cl) thus obtained did not include triacontane, a homo-coupling product of pentadecylmagnesium chloride (triacontane was below the GC detection limit).

The following is the spectrum data of 1-chloro-2-methyloctadecane (9: $X^7$ = Cl) thus prepared.

Nuclear magnetic resonance spectrum: $^1$H-NMR (500 MHz, $CDCl_3$): δ = 0.88 (3H, t, J = 6.9 Hz), 1.00 (3H, J = 6.9 Hz), 1.20-1.50 (30H, m), 1.76-1.84 (1H, m), 3.48 (1H, dd, J = 10.5 Hz, 5.4 Hz), 3.40 (1H, dd, J = 10.5 Hz, 6.5 Hz); $^{13}$C-NMR (125 MHz, $CDCl_3$): δ = 14.11, 17.77, 22.69, 26.84, 29.37, 29.59, 29.63, 29.66, 29.70, 29.75, 31.93, 33.97, 35.52, 51.28.

Mass spectrum: EI-mass spectrum (70 eV): m/z 302 ($M^+$), 253, 197, 188, 169, 153, 141, 127, 113, 105, 99, 85, 71, 57, 41.

Infrared absorption spectrum: (D-ATR): ν = 2924, 2853, 1465, 1378, 722, 688.

Example 3: Preparation of 1-chloro-7-methyltricosane (1: $X^1$ = Cl)

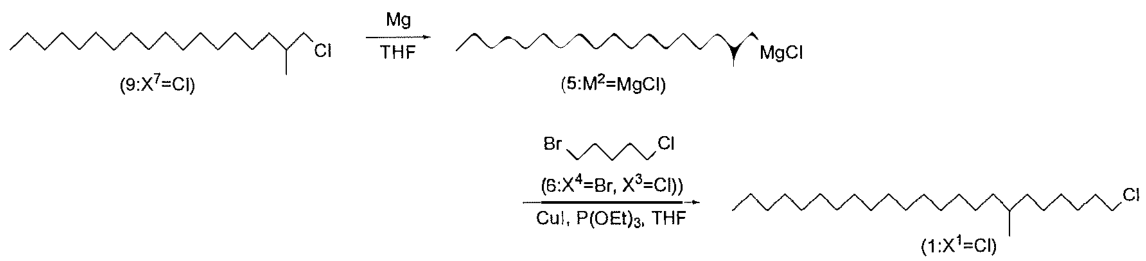


Magnesium (59.35 g, 2.44 gram atoms) and tetrahydrofuran (930.40 g) were placed in a reactor at a room temperature and stirred for 35 minutes at 60 to 65°C. l-Chloro-2-methyloctadecane (9: $X^7$ = Cl) (712.40 g, 2.33 mol, purity 98.92%) prepared in Example 2 was then added dropwise to the reactor at 60 to 75°C. After the completion of the dropwise addition, the reaction mixture was stirred for 2 hours at 75 to 80°C to obtain 2-methyloctadecylmagnesium chloride (5: $M^2$ = MgCl).

Next, cuprous iodide (4.43 g, 0.023 mol), triethyl phosphite (9.28 g, 0.056 mol), tetrahydrofuran (465.20 g), and 1-bromo-5-chloropentane (6: $X^4$ = Br, $X^3$ = Cl) (409.88 g, 2.21 mol, purity 100%) were placed in another reactor, and the aforesaid pentadecylmagnesium chloride that was prepared was added dropwise at 15 to 25°C. After the completion of the dropwise addition, the reaction mixture was stirred for 1.5 hours at 20 to 30°C. An aqueous solution of ammonium chloride (prepared from ammonium chloride (23.26 g) and water (640.81 g)), 20% by mass hydrochloric acid (22.27 g), and an aqueous solution (11.83 g) of 25% by mass sodium hydroxide were then added to the reaction mixture, followed by phase separation. The organic layer thus obtained was concentrated at a reduced pressure, and the resulting concentrate was distilled at a reduced pressure to obtain 1-chloro-7-methyltricosane (1: $X^1$ = Cl) (708.34 g, 1.85 mol, purity 97.18%, b.p. = 183.1 to 196.2°C/0.044 kPa (0.33 mmHg)) as a liquid with a yield of 83.50%. 1-Chloro-7-methyltricosane (1: $X^1$ = Cl) thus obtained did not include 17,20-dimethylhexatriacontane, a homo-coupling product of 2-methyloctadecylmagnesium chloride (5: $M^2$ = MgCl) (17,20-dimethylhexatriacontane was below the GC detection limit).

The following is the spectrum data of 1-chloro-7-methyltricosane (1: $X^1$ = Cl) thus prepared.

Nuclear magnetic resonance spectrum: $^1$H-NMR (500 MHz, $CDCl_3$): δ = 0.84 (3H, J = 6.5 Hz), 0.88 (3H, t, J = 6.9 Hz), 1.04-1.12 (2H, m), 1.20-1.33 (35H, m), 1.38-1.47 (2H, m), 1.77 (2H, tt, J = 6.9 Hz, 6.9 Hz), 3.53 (2H, t, J = 6.9 Hz); $^{13}$C-NMR (125 MHz, $CDCl_3$): δ = 14.11, 19.68, 22.70, 26.90, 26.93, 27.09, 29.25, 29.37, 29.66, 29.71, 29.74, 30.02, 31.93, 32.68, 32.71, 36.95, 37.06, 45.17.

Mass spectrum: EI-mass spectrum (70 eV): m/z 357 ($M^+$-14), 253, 224, 197, 169, 147, 111, 99, 85, 71, 57, 43, 29.

Infrared absorption spectrum: (D-ATR): ν = 2924, 2853, 1465, 1377, 1304, 723, 656.

Example 4: Preparation of 17-methylpentatriacontane (4: *n* = 11)

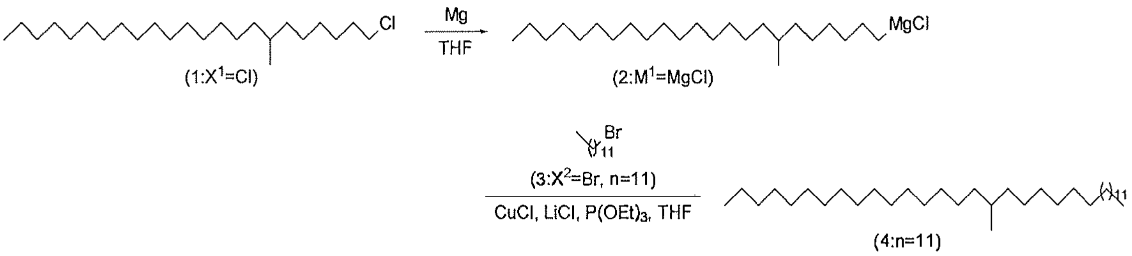


Magnesium (3.31 g, 0.14 gram atoms) and tetrahydrofuran (86.5 g) were placed in a reactor at a room temperature and stirred for 28 minutes at 60 to 65°C. 1-Chloro-7-methyltricosane (1: $X^1$ = Cl) (49.87 g, 0.13 mol, purity 97.18%) prepared in Example 3 was then added dropwise to the reactor at 60 to 75°C. After the completion of the dropwise addition, the reaction mixture was stirred for 3.5 hours at 75 to 80°C to obtain 7-methyltricosylmagnesium chloride (2: $M^1$ = MgCl).

Next, cuprous chloride (0.15 g, 0.0015 mol), triethyl phosphite (1.46 g, 0.0088 mol), lithium chloride (0.10 g, 0.0024 mol), tetrahydrofuran (100.00 g), and 1-bromododecane (3: $X^2$ = Br, *n* = 11) (32.38 g, 0.13 mol) were placed in another reactor, and the aforesaid 7-methyltricosylmagnesium chloride (2: $M^1$ = MgCl) that was prepared was added dropwise at 15 to 25°C. After the completion of the dropwise addition, the reaction mixture was stirred for 2.5 hours at 20 to 30°C. An aqueous solution of acetic acid (prepared from acetic acid (1.30 g) and water (35.79 g)), 20% by mass hydrochloric acid (2.72 g), and an aqueous solution (2.72 g) of 25% by mass sodium hydroxide were then added to the reaction mixture, followed by phase separation. The organic layer thus obtained was subjected as such to distillation at a reduced pressure to separate tetracosane. The resulting concentrate was then purified by silica gel column chromatography (hexane:ethyl acetate = 100:0) to obtain 17-methylpentatriacontane (4: *n* = 11) (56.51 g, 0.10 mol, purity 93.48%) as a solid with a yield of 80.23%. 17-Methylpentatriacontane *(4: n* = 11) thus obtained did not include 17,30-dimethylhexatetracontane, a homo-coupling product of 7-methyltricosylmagnesium chloride (2: $M^1$ = MgCl) (17,30-dimethylhexatetracontane was below the GC detection limit). Although purification by column chromatography was carried out in this Example, purification may be carried out by distillation and/or recrystallization because there were no impurities to be separated.

The following is the spectrum data of 17-methylpentatriacontane (4: *n* = 11) thus prepared.

Nuclear magnetic resonance spectrum: $^1$H-NMR (500 MHz, $CDCl_3$): δ = 0.84 (3H, J = 6.9 Hz), 0.88 (6H, t-like, J = 6.9 Hz),1.18-1.33 (65H, m); $^{13}$C-NMR (125 MHz, $CDCl_3$): δ = 14.13, 19.73, 22.67, 22.71, 27.11, 29.38, 29.68, 29.73, 29.76, 30.05, 31.95, 32.76, 37.11.

Mass spectrum: EI-mass spectrum (70 eV): m/z 492 ($M^+$-14), 280, 252, 224, 197, 183, 169, 155, 141, 127, 113, 99, 85, 71, 57, 43, 29.

Infrared absorption spectrum: (D-ATR): ν = 2956, 2917, 2850, 1470, 1377, 720.

Example 5: Preparation of 17-methylheptatriacontane (4: *n* = 13)

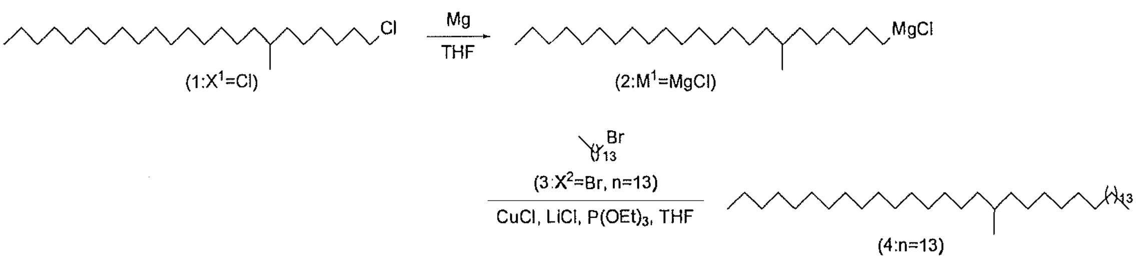


Magnesium (3.31 g, 0.14 gram atoms) and tetrahydrofuran (86.5 g) were placed in a reactor at a room temperature and stirred for 19 minutes at 60 to 65°C. 1-Chloro-7-methyltricosane (1: $X^1$ = Cl) (49.87 g, 0.13 mol, purity 97.18%) prepared in Example 3 was then added dropwise to the reactor at 60 to 75°C. After the completion of the dropwise addition, the reaction mixture was stirred for 5 hours at 75 to 80°C to obtain 7-methyltricosylmagnesium chloride (2: $M^1$ = MgCl).

Next, cuprous chloride (0.15 g, 0.0015 mol), triethyl phosphite (1.46 g, 0.0088 mol), lithium chloride (0.10 g, 0.0024 mol), tetrahydrofuran (100.00 g), and 1-bromotetradecane (3: $X^2$ = Br, *n* = 13) (36.02 g, 0.13 mol) were placed in another reactor, and the aforesaid 7-methyltricosylmagnesium chloride (2: $M^1$ = MgCl) that was prepared was added dropwise at 15 to 25°C. After the completion of the dropwise addition, the reaction mixture was stirred for 2 hours at 20 to 30°C. An aqueous solution of acetic acid (prepared from acetic acid (1.30 g) and water (35.79 g)), 20% by mass hydrochloric acid (2.72 g), and an aqueous solution (2.72 g) of 25% by mass sodium hydroxide were then added to the reaction mixture, followed by phase separation. The organic layer thus obtained was subjected as such to distillation at a reduced pressure to separate octacosane.
The resulting concentrate was then purified by silica gel column chromatography (hexane:ethyl acetate = 100:0) to obtain 17-methylheptatriacontane (4: *n* = 13) (54.82 g, 0.094 mol, purity 91.51%) as a solid with a yield of 72.18%. 17-Methylheptatriacontane *(4: n* = 13) thus obtained did not include 17,30-dimethylhexatetracontane, a homo-coupling product of 7-methyltricosylmagnesium chloride (2: $M^1$ = MgCl) (17,30-dimethylhexatetracontane was below the GC detection limit). Although purification by column chromatography was carried out in this Example, purification may be carried out by distillation and/or recrystallization because there were no impurities to be separated.

The following is the spectrum data of 17-methylheptatriacontane (4: *n* = 13) thus prepared.

Nuclear magnetic resonance spectrum: $^1$H-NMR (500 MHz, $CDCl_3$): $\delta$ = 0.84 (3H, J = 6.5 Hz), 0.88 (6H, t, J = 6.9 Hz), 1.03-1.12 (2H, m), 1.20-1.33 (67H, m); $^{13}$C-NMR (125 MHz, $CDCl_3$): $\delta$ = 14.12, 19.73, 22.71, 27.10, 29.38, 29.68, 29.72, 29.75, 30.05, 31.94, 32.75, 37.11.

Mass spectrum: EI-mass spectrum (70 eV): m/z 519 ($M^+$-1), 463, 393, 351, 308, 252, 183, 169, 155, 141, 71, 57.

Infrared absorption spectrum: (D-ATR): ν =2958, 2918, 2850, 1473, 1464, 1377, 729, 719.

Example 6: Preparation of 1-bromo-7-methyltricosane (1: $X^1$ = Br)

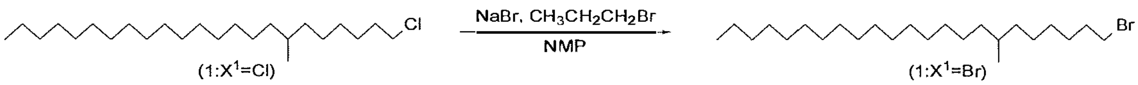


1-Chloro-7-methyltricosane (1: $X^1$ = Cl) (10.00 g, 0.26 mol, purity 97.18%), 1-bromopropane ($CH_3CH_2CH_2Br$) (19.02 g, 0.15 mol), sodium bromide (NaBr) (0.53 g, 0.0052 mol), and N-methyl-2-pyrrolidone (NMP) (19.00 g) were placed in a reactor at a room temperature and heated to 110 to 120°C. 1-Chloropropane produced in the reaction was removed from the reaction system by distillation. After the conversion was confirmed to be 99% by GC, the reaction mixture was cooled to 25°C. Water (35.16 g) and hexane (10.31 g) were then added to the reaction mixture, followed by phase separation. The organic layer thus obtained was concentrated, and then the resulting concentrate was purified by silica gel column chromatography (hexane:ethyl acetate = 50:1) to obtain 1-bromo-7-methyltricosane (1: $X^1$ = Br) (10.93 g, 0.26 mol, purity 100%) as a liquid with a yield of 100%.

The following is the spectrum data of 1-bromo-7-methyltricosane (1: $X^1$ = Br) thus prepared.

Nuclear magnetic resonance spectrum: $^1$H-NMR (500 MHz, $CDCl_3$): $\delta$ = 0.84 (3H, J = 6.5 Hz), 0.88 (3H, t, J = 6.9 Hz), 1.03-1.14 (2H, m), 1.18-1.36 (35H, m), 1.39-1.47 (2H, m), 1.86 (2H, tt, J = 6.9 Hz, 6.9 Hz), 3.41 (2H, t, J= 6.9 Hz); $^{13}$C-NMR (125 MHz, $CDCl_3$): $\delta$ = 14.11, 19.68, 22.70, 26.87, 27.09, 28.23, 29.13, 29.37, 29.66, 29.71, 29.74, 30.02, 31.93, 32.71, 32.86, 34.02, 36.94, 37.06.

Mass spectrum: EI-mass spectrum (70 eV): m/z 403 ($M^+$-13), 337, 253, 191, 169, 155, 127, 111, 99, 85, 71, 57, 43, 29.

Infrared absorption spectrum: (D-ATR): ν = 2923, 2853, 1465, 1377, 1259, 722, 648, 566.

## Claims

**1.** A 1-halo-7-methyltricosane compound of the following general formula (1):

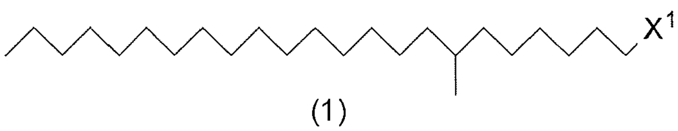

(1)

wherein $X^1$ represents a halogen atom.

**2.** A process for preparing a 17-methylalkane compound of the following general formula (4):

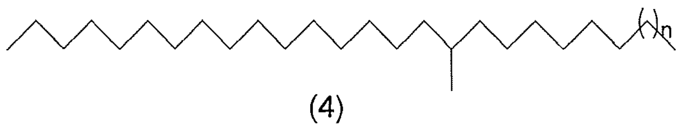

(4)

wherein n represents an integer of 11 to 13,
the process comprising
converting a 1-halo-7-methyltricosane compound of the following general formula (1):

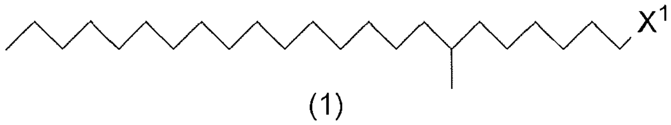

(1)

wherein $X^1$ represents a halogen atom,
into a nucleophilic reagent, 7-methyltricosyl compound, of the following general formula (2):

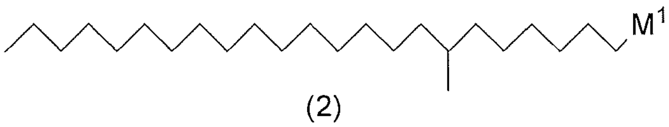

(2)

wherein $M^1$ represents Li, $MgZ^1$, $CuZ^1$, or $CuLiZ^1$, and $Z^1$ represents a halogen atom or a 7-methyltricosyl group, subsequently subjecting the aforesaid nucleophilic reagent, 7-methyltricosyl compound (2), to a coupling reaction with an electrophilic alkyl reagent of the following general formula (3):

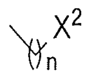

(3)

wherein $X^2$ represents a halogen atom or a *p*-toluenesulfonyloxy group, and n is as defined above,
to form the aforesaid 17-methylalkane compound (4).

**3.** The process for preparing the aforesaid 17-methylalkane compound (4) according to claim 2,

the process further comprising
subjecting a nucleophilic reagent, 2-methyloctadecyl compound, of the following general formula (5):

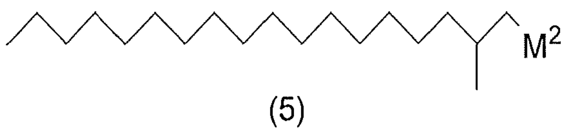

(5)

wherein $M^2$ represents Li, $MgZ^2$, $CuZ^2$, or $CuLiZ^2$, and $Z^2$ represents a halogen atom or a 2-methyloctadecyl group,
to a coupling reaction with a 1,5-dihalopentane compound of the following general formula (6):

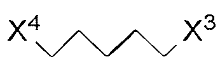

(6)

wherein $X^3$ and $X^4$ represent, independently of each other, a halogen atom,
to form the aforesaid 1-halo-7-methyltricosane compound (1).

**4.** The process for preparing the aforesaid 17-methylalkane compound (4) according to claim 3,

the process further comprising
subjecting a nucleophilic reagent, pentadecyl compound, of the following general formula (7):

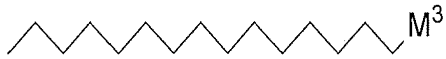

(7)

wherein $M^3$ represents Li, $MgZ^3$, $CuZ^3$, or $CuLiZ^3$, and $Z^3$ represents a halogen atom or a pentadecyl group, to a coupling reaction with a 1,3-dihalo-2-methylpropane compound of the following general formula (8):

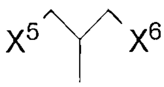

(8)

wherein $X^5$ and $X^6$ represent, independently of each other, a halogen atom,
to form a 1-halo-2-methyloctadecane compound of the following general formula (9):

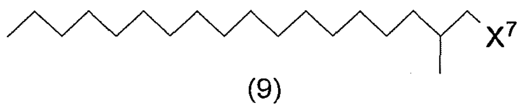

(9)

wherein $X^7$ represents a halogen atom, and
preparing the aforesaid nucleophilic reagent, 2-methyloctadecyl compound (5) from the aforesaid 1-halo-2-methyloctadecane compound (9).

**5.** A process for preparing a 1-halo-7-methyltricosane compound of the following general formula (1):

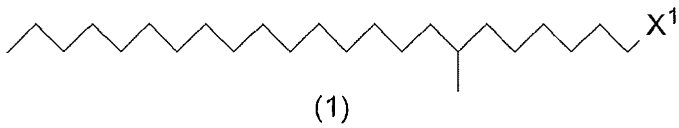

(1)

wherein $X^1$ represents a halogen atom,
the process comprising
subjecting a nucleophilic reagent, 2-methyloctadecyl compound, of the following general formula (5):

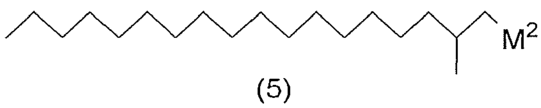

(5)

wherein $M^2$ represents Li, $MgZ^2$, $CuZ^2$, or $CuLiZ^2$, and $Z^2$ represents a halogen atom or a 2-methyloctadecyl group,
to a coupling reaction with a 1,5-dihalopentane compound of the following general formula (6):

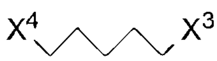

(6)

wherein $X^3$ and $X^4$ represent, independently of each other, a halogen atom,
to form the aforesaid 1-halo-7-methyltricosane compound (1).

**6.** The process for preparing the aforesaid 1-halo-7-methyltricosane compound (1) according to claim 5,

the process further comprising
subjecting a nucleophilic reagent, pentadecyl compound, of the following general formula (7):

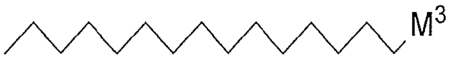

(7)

wherein $M^3$ represents Li, $MgZ^3$, $CuZ^3$, or $CuLiZ^3$, and $Z^3$ represents a halogen atom or a pentadecyl group, to a coupling reaction with a 1,3-dihalo-2-methylpropane compound of the following general formula (8):

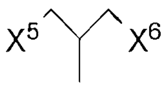

(8)

wherein $X^5$ and $X^6$ represent, independently of each other, a halogen atom,
to form a 1-halo-2-methyloctadecane compound of the following general formula (9):

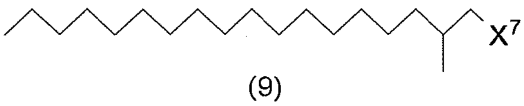

(9)

wherein $X^7$ represents a halogen atom, and
preparing the aforesaid nucleophilic reagent, 2-methyloctadecyl compound (5) from the aforesaid 1-halo-2-methyloctadecane compound (9).

**Patentansprüche**

**1.** Eine 1-Halogen-7-methyltricosanverbindung der folgenden allgemeinen Formel (1):

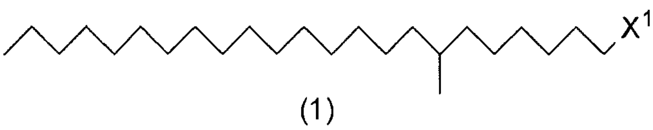

(1)

wobei $X^1$ ein Halogenatom darstellt.

**2.** Ein Verfahren zur Herstellung einer 17-Methylalkanverbindung der folgenden allgemeinen Formel (4):

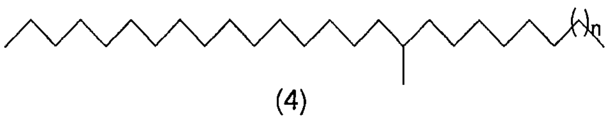

(4)

wobei n eine ganze Zahl von 11 bis 13 darstellt,
wobei das Verfahren umfasst
Umwandeln einer 1-Halogen-7-methyltricosanverbindung der folgenden allgemeinen Formel (1):

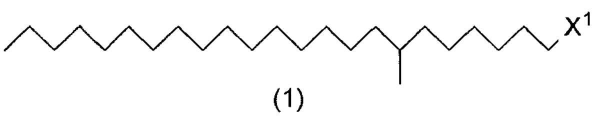

(1)

wobei $X^1$ ein Halogenatom darstellt,
in ein nukleophiles Reagenz, eine 7-Methyltricosylverbindung, der folgenden allgemeinen Formel (2):

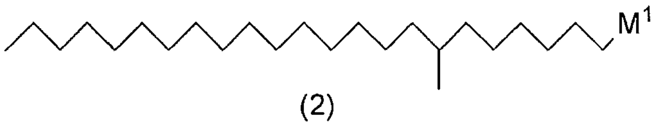

(2)

wobei $M^1$ für Li, $MgZ^1$, $CuZ^1$ oder $CuLiZ^1$ steht und $Z^1$ ein Halogenatom oder eine 7-Methyltricosylgruppe darstellt,
anschließendes Unterziehen der vorstehenden nukleophilen Reagenz, einer 7-Methyltricosylverbindung (2), einer Kupplungsreaktion mit einem elektrophilen Alkylreagenz der folgenden allgemeinen Formel (3):

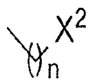

(3)

wobei $X^2$ ein Halogenatom oder eine p-Toluolsulfonyloxygruppe darstellt und n wie vorstehend definiert ist, um die vorstehende 17-Methylalkanverbindung (4) zu bilden.

**3.** Das Verfahren zur Herstellung der vorstehenden 17-Methylalkanverbindung (4) nach Anspruch 2,

wobei das Verfahren ferner umfasst
Unterziehen einer nukleophilen Reagenz, einer 2-Methyloctadecylverbindung, der folgenden allgemeinen Formel (5):

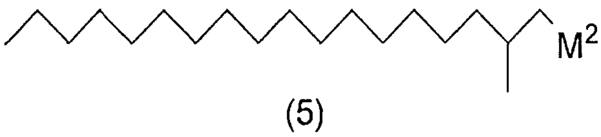

(5)

wobei $M^2$ für Li, $MgZ^2$, $CuZ^2$ oder $CuLiZ^2$ steht und $Z^2$ ein Halogenatom oder eine 2-Methyloctadecylgruppe darstellt,
einer Kupplungsreaktion mit einer 1,5-Dihalogenpentanverbindung der folgenden allgemeinen Formel (6):

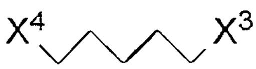

(6)

wobei $X^3$ und $X^4$ unabhängig voneinander ein Halogenatom darstellen,
um die vorstehende 1-Halogen-7-methyltricosanverbindung (1) zu bilden.

**4.** Das Verfahren zur Herstellung der vorstehenden 17-Methylalkanverbindung (4) nach Anspruch 3,

wobei das Verfahren ferner umfasst
Unterziehen einer nukleophilen Reagenz, einer Pentadecylverbindung, der folgenden allgemeinen Formel (7):

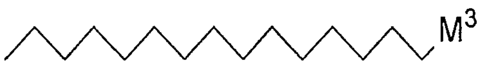

(7)

wobei $M^3$ für Li, $MgZ^3$, $CuZ^3$ oder $CuLiZ^3$ steht und $Z^3$ ein Halogenatom oder eine Pentadecylgruppe darstellt,
einer Kupplungsreaktion mit einer 1,3-Dihalogen-2-methylpropanverbindung der folgenden allgemeinen Formel

(8):

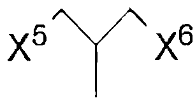

(8)

wobei $X^5$ und $X^6$ unabhängig voneinander ein Halogenatom darstellen, um eine 1-Halogen-2-methyloctadecanverbindung der folgenden allgemeinen Formel (9) zu bilden:

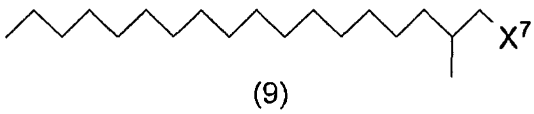

(9)

wobei $X^7$ ein Halogenatom darstellt, und Herstellen der vorstehenden nukleophilen Reagenz, einer 2-Methyloctadecylverbindung (5), aus der vorstehenden 1-Halogen-2-methyloctadecanverbindung (9).

**5.** Ein Verfahren zur Herstellung einer 1-Halogen-7-methyltricosanverbindung der folgenden allgemeinen Formel (1):

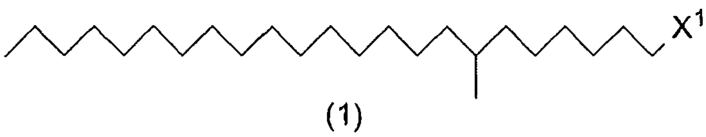

(1)

wobei $X^1$ ein Halogenatom darstellt,
wobei das Verfahren umfasst
Unterziehen einer nukleophilen Reagenz, einer 2-Methyloctadecylverbindung, der folgenden allgemeinen Formel (5):

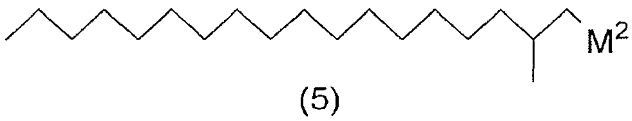

(5)

wobei $M^2$ für Li, $MgZ^2$, $CuZ^2$ oder $CuLiZ^2$ steht und $Z^2$ ein Halogenatom oder eine 2-Methyloctadecylgruppe darstellt,
einer Kupplungsreaktion mit einer 1,5-Dihalogenpentanverbindung der folgenden allgemeinen Formel (6):

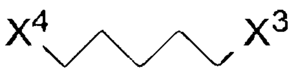

(6)

wobei $X^3$ und $X^4$ unabhängig voneinander ein Halogenatom darstellen, um die vorstehende 1-Halogen-7-methyltricosanverbindung (1) zu bilden.

**6.** Das Verfahren zur Herstellung der vorstehenden 1-Halogen-7-methyltricosanverbindung (1) nach Anspruch 5,

wobei das Verfahren ferner umfasst
Unterziehen einer nukleophilen Reagenz, einer Pentadecylverbindung, der folgenden allgemeinen Formel (7):

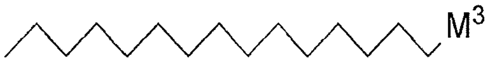

(7)

wobei $M^3$ für Li, $MgZ^3$, $CuZ^3$ oder $CuLiZ^3$ steht und $Z^3$ ein Halogenatom oder eine Pentadecylgruppe darstellt, einer Kupplungsreaktion mit einer 1,3-Dihalogen-2-methylpropanverbindung der folgenden allgemeinen Formel (8):

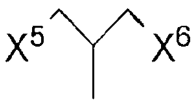

(8)

wobei $X^5$ und $X^6$ unabhängig voneinander ein Halogenatom darstellen,
um eine 1-Halogen-2-methyloctadecanverbindung der folgenden allgemeinen Formel (9) zu bilden:

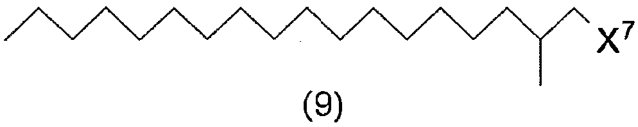

(9)

wobei $X^7$ ein Halogenatom darstellt, und
Herstellen der vorstehenden nukleophilen Reagenz, einer 2-Methyloctadecylverbindung (5), aus der vorstehenden 1-Halogen-2-methyloctadecanverbindung (9).

## Revendications

**1.** Composé de 1-halo-7-méthyltricosane de formule générale (1) suivante :

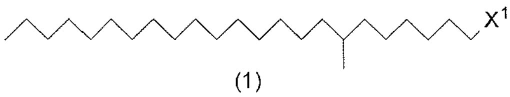

(1)

dans laquelle $X^1$ représente un atome d'halogène.

**2.** Procédé de préparation d'un composé de 17-méthylalcane de formule générale (4) suivante :

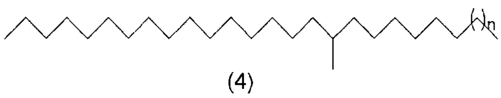

(4)

dans laquelle n représente un entier de 11 à 13,
le procédé comprenant
la conversion d'un composé de 1-halo-7-méthyltricosane de formule générale (1) suivante :

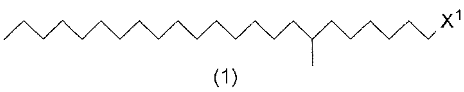

(1)

dans laquelle $X^1$ représente un atome d'halogène,
en un réactif nucléophile, le composé de 7-méthyltricosyle, de formule générale (2) suivante :

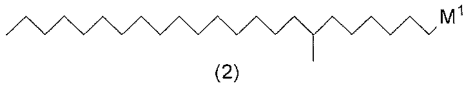

(2)

dans laquelle $M^1$ représente Li, $MgZ^1$, $CuZ^1$, ou $CuLiZ^1$, et $Z^1$ représente un atome d'halogène ou un groupe 7-méthyltricosylyle,
puis la soumission du réactif nucléophile susmentionné, le composé de 7-méthyltricosyle (2), à une réaction de couplage avec un réactif alkyle électrophile de formule générale (3) suivante :

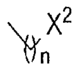

(3)

dans laquelle $X^2$ représente un atome d'halogène ou un groupe p-toluènesulfonyloxy, et n est défini comme ci-dessus,
pour former le composé de 17-méthylalcane (4) susmentionné.

**3.** Procédé de préparation du composé de 17-méthylalcane (4) susmentionné selon la revendication 2,

le procédé comprenant en outre
la soumission d'un réactif nucléophile, le composé de 2-méthyloctadécyle, de formule générale (5) suivante :

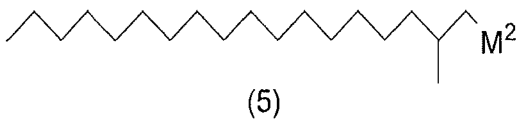

(5)

dans laquelle $M^2$ représente Li, $MgZ^2$, $CuZ^2$, ou $CuLiZ^2$, et $Z^2$ représente un atome d'halogène ou un groupe 2-méthyloctadécyle,
à une réaction de couplage avec un composé de 1,5-dihalopentane de formule générale (6) suivante :

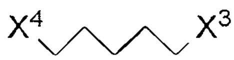

(6)

dans laquelle $X^3$ et $X^4$ représentent, indépendamment l'un de l'autre, un atome d'halogène, pour former le composé de 1-halo-7-méthyltricosane (1) susmentionné.

**4.** Procédé de préparation du composé de 17-méthylalcane (4) susmentionné selon la revendication 3,

le procédé comprenant en outre
la soumission d'un réactif nucléophile, le composé de pentadécyle, de formule générale (7) suivante :

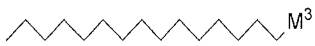

(7)

dans laquelle $M^3$ représente Li, $MgZ^3$, $CuZ^3$, ou $CuLiZ^3$, et $Z^3$ représente un atome d'halogène ou un groupe pentadécyle, à une réaction de couplage avec un composé de 1,3-dihalo-2-méthylpropane de formule générale (8) suivante :

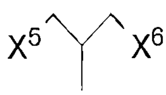

(8)

dans laquelle $X^5$ et $X^6$ représentent, indépendamment l'un de l'autre, un atome d'halogène, afin de former un composé de 1-halo-2-méthyloctadécane de formule générale (9) suivante :

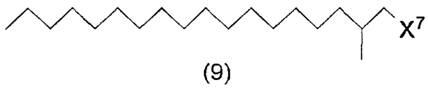

(9)

dans laquelle $X^7$ représente un atome d'halogène, et
la préparation du réactif nucléophile susmentionné, le composé de 2-méthyloctadécyle (5) à partir du composé de 1-halo-2-méthyloctadécane (9) susmentionné.

**5.** Procédé de préparation d'un composé de 1-halo-7-méthyltricosane de formule générale (1) suivante :

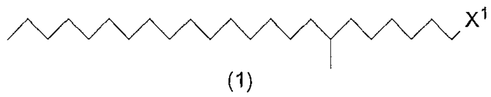

(1)

dans laquelle $X^1$ représente un atome d'halogène,
le procédé comprenant
la soumission d'un réactif nucléophile, le composé de 2-méthyloctadécyle, de formule générale (5) suivante :

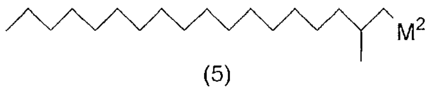

(5)

dans laquelle $M^2$ représente Li, $MgZ^2$, $CuZ^2$, ou $CuLiZ^2$, et $Z^2$ représente un atome d'halogène ou un groupe 2-méthyloctadécyle,
à une réaction de couplage avec un composé de 1,5-dihalopentane de formule générale (6) suivante :

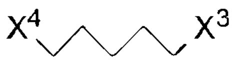

(6)

dans laquelle $X^3$ et $X^4$ représentent, indépendamment l'un de l'autre, un atome d'halogène,
pour former le composé de 1-halo-7-méthyltricosane (1) susmentionné.

**6.** Procédé de préparation du composé de 1-halo-7-méthyltricosane (1) susmentionné selon la revendication 5, le procédé comprenant en outre

la soumission d'un réactif nucléophile, le composé de pentadécyle, de formule générale (7) suivante :

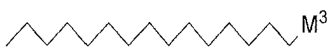

(7)

dans laquelle $M^3$ représente Li, $MgZ^3$, $CuZ^3$, ou $CuLiZ^3$, et $Z^3$ représente un atome d'halogène ou un groupe pentadécyle, à une réaction de couplage avec un composé de 1,3-dihalo-2-méthylpropane de formule générale (8) suivante :

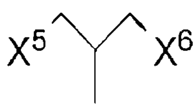

(8)

dans laquelle $X^5$ et $X^6$ représentent, indépendamment l'un de l'autre, un atome d'halogène,

pour former un composé de 1-halo-2-méthyloctadécane de formule générale (9) suivante :

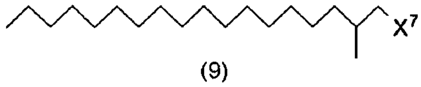

(9)

dans laquelle $X^7$ représente un atome d'halogène, et
la préparation du réactif nucléophile susmentionné, le composé de 2-méthyloctadécyle (5) à partir du composé de 1-halo-2-méthyloctadécane (9) susmentionné.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NEIL D TSUTSUI et al.** *BMC Biology*, 2009, vol. 7, 71
- **NEIL D TSUTSUI et al.** *J. Chem. Ecol.*, 2010, vol. 36, 751-758
- **E. SUNAMURA et al.** *Insectes Sociaux*, 2009, vol. 56, 143-147
- **DENNIS H. BURNS et al.** *J. Am. Chem. Soc.*, 1997, vol. 119, 2125-2133